# DEMANDE DE BREVET EUROPEEN

(11) **EP 1 358 868 A2**
(43) Date de publication de la demande: **05.11.2003**
(21) Numéro de dépôt: 03290990.5
(22) Date de dépôt: 23.04.2003
(51) Int. Cl.: A61K 7/06

(54) **Composition cosmétique pour favoriser la pousse et/ou empecher ou retarder la chute des cheveux**

(30) Priorité: 23.04.2002 FR 0205067; 28.10.2002 FR 0213461
(71) Demandeur: L'OREAL, 75008 Paris (FR)
(72) Inventeur: Michelet, Jean-Francois, 94000 Creteil (FR); Bernard, Bruno, 92200 Neuilly sur Seine (FR); Rozot, Roger, 77400 Lagny/Marne (FR); Boulle, Christophe, Lagny s/Marne 77400 (FR)
(74) Mandataire: Allab, Myriam

(57) **Abrégé**

L'invention concerne une composition cosmétique contenant au moins un inhibiteur de la 15-hydroxy prostaglandine déshydrogénase et des excipients cosmétologiquement acceptables. Elle concerne également un procédé de traitement cosmétique pour favoriser la pousse et/ou empêcher ou retarder la chute des cheveux, ainsi que l'utilisation d'un inhibiteur de 15-hydroxy prostaglandine déshydrogénase pour la préparation d'une composition destinée à lutter contre la chute des cheveux et/ou à favoriser la repousse des cheveux.

## Description

La présente invention concerne l'utilisation de composés pour la préparation de compositions destinées à induire ou stimuler la croissance des fibres kératiniques et en particulier des cheveux et des cils humains. L' invention se rapporte à de nouveaux composés pour lutter contre la chute des cheveux et/ou favoriser la repousse des cheveux. Elle se rapporte également à un procédé de traitement cosmétique pour lutter contre la chute des fibres kératiniques, telles que les cheveux ou les cils et/ou favoriser la croissance des fibres kératiniques, notamment la pousse ou la repousse des cheveux, en particulier pour maintenir et/ou augmenter la densité capillaire chez l'homme.

Chez l'être humain, la croissance des cheveux et leur renouvellement sont principalement déterminés par l'activité des follicules pileux et de leur environnement cutané et/ou conjonctif . Leur activité est cyclique et comporte essentiellement trois phases, à savoir la phase anagène, la phase catagène et la phase télogène.

A la phase anagène active ou phase de croissance, qui dure plusieurs années et au cours de laquelle les cheveux s'allongent, succède une phase catagène très courte et transitoire qui dure quelques semaines. Au cours de cette phase, le cheveu subit une involution, le follicule s'atrophie et son implantation dermique apparaît de plus en plus haute.
La phase terminale, dite télogène, qui dure quelques mois, correspond à une période de repos du follicule et le cheveu finit par tomber. Après cette phase de repos un nouveau follicule est régénéré, sur place, et un nouveau cycle recommence.

La chevelure se renouvelle donc en permanence, et sur les 150 000 cheveux environ que comporte une chevelure, à chaque instant, 10% d'entre eux environ sont au repos et seront donc remplacés en quelques mois.

La chute ou perte naturelle des cheveux peut être estimée, en moyenne, à quelques cent cheveux par jour pour un état physiologique normal. Ce processus de renouvellement physique permanent subit une évolution naturelle au cours du vieillissement, les cheveux deviennent plus fins, leurs cycles plus courts

En outre, différentes causes peuvent entraîner une perte temporaire ou définitive, des cheveux.

Il peut s'agir de chute et d'altération des cheveux au décours d'une grossesse (effluvium post partum), au cours d'états de dénutrition ou de déséquilibres alimentaires, ou encore au cours d'états d'asthénie ou de dysfonctionnement hormonal comme cela peut être le cas au cours ou au décours de la ménopause. Il peut également s'agir de chute ou d'altérations des cheveux en relation avec des phénomènes saisonniers.

Il peut également s'agir d'une alopécie qui est essentiellement due à une perturbation du renouvellement capillaire qui entraîne, dans un premier temps, l'accélération de la fréquence des cycles aux dépens de la qualité des cheveux puis de leur quantité. Les cycles de croissance successifs aboutissent à des cheveux de plus en plus fins et de plus en plus courts, se transformant peu à peu en un duvet non pigmenté. Des zones sont touchées préférentiellement, notamment les golfes temporaux ou frontaux chez l'homme, et chez les femmes, on constate une alopécie diffuse du vertex.

Le terme alopécie recouvre aussi toute une famille d'atteintes du follicule pileux ayant pour conséquence finale la perte définitive partielle ou générale des cheveux.
Il s'agit plus particulièrement de l'alopécie androgénique. Dans un nombre important de cas, la chute précoce des cheveux survient chez des sujets prédisposés génétiquement, il s'agit alors d'alopécie andro-chrono-génétique ; cette forme d'alopécie concerne notamment les hommes.

Il est connu, par ailleurs, que certains facteurs tels qu'un déséquilibre hormonal, un stress physiologique, la malnutrition, peuvent accentuer le phénomène.

Dans certaines dermatoses du cuir chevelu à caractéristique inflammatoire, telles que par exemple le psoriasis ou les dermatites séborrhéiques, la chute des cheveux peut être fortement accentuée ou entraîner des cycles des follicules fortement perturbés.

On recherche depuis de nombreuses années, dans l'industrie cosmétique ou pharmaceutique, des compositions permettant de supprimer ou de réduire l'alopécie, et notamment d'induire ou de stimuler la croissance des cheveux ou de diminuer leur chute.

Dans cette optique, on a déjà proposé un grand nombre de compositions comprenant des actifs très divers, comme par exemple le 2,4-diamino 6-piperidinopyrimidine 3-oxyde ou "Minoxidil" décrit dans les brevets US 4 139 619 et US 4 596 812 ou encore ses nombreux dérivés comme ceux décrits par exemple dans les demandes de brevet EP 0353123, EP 0356271, EP 0408442, EP 0522964, EP 0420707, EP 0459890, EP 0519819.

Des études cliniques ont démontré que des analogues de PGF2 α avaient la propriété de provoquer la croissance de poils et de cils chez l'homme et chez l'animal (Murray A and Johnstone MD, 1997. *Am J Opht*, **124**(4), 544-547). Chez l'homme, des essais réalisés sur le cuir chevelu ont montré qu'un analogue de prostaglandine E2 (le viprostol) avait la propriété d'augmenter la densité capillaire (Roenigk HH., 1988. *Clinic Dermatol*, **6(4),** 119-121).
Le brevet WO 98/33497 décrit des compositions pharmaceutiques contenant des prostaglandines ou des dérivés de prostaglandines destinées à lutter contre la chute des cheveux chez l'homme. Les prostaglandines du type A2, F2α et E2 sont préférées.

Cependant, les prostaglandines sont des molécules au temps de demi-vie biologique très court et agissant de façon autocrine ou paracrine, ceci traduisant un caractère labile dû à un métabolisme local très actif des prostaglandines (Narumiya S et *al*., 1999, *Physiol Rev*, **79(4),** 1193-1226).

Il apparaît donc comme important, pour maintenir et/ou augmenter la densité capillaire chez l'homme de préserver les réserves endogènes de PGF2 α comme de PGE2 des différents compartiments du follicule pileux ou de son environnement cutané proche.

Une solution donnant de bons résultats est l'administration de composés inhibiteurs de lipoxygénase et/ou inducteurs de la cyclo-oxygénase en vue de favoriser la croissance des cheveux; une hypothèse est que l'administration de tels composés oriente le métabolisme des acides gras vers la synthèse endogène de prostaglandines de préférence à d'autres voies.

Toutefois, pour améliorer encore les résultats, il serait souhaitable de pouvoir prolonger l'activité des prostaglandines impliquées dans la croissance et le maintien de l'activité du follicule pileux.

Il est par ailleurs bien connu que les programmes de différenciation des kératinocytes de l'épiderme et du follicule pileux sont clairement différents. Ainsi, il est connu que les kératines de la tige pilaire représente une famille (Langbein et al., 2001, J.Biol.Chem. 276 :35123-35132) distincte de celle exprimée dans l'épiderme, que les marqueurs de différenciation telle que les kératines K1 et K10 ne sont pas exprimés dans le follicule pileux et en particulier dans la gaine externe (Lenoir et al., 1988, Dev. Biol. 130: 610-620), que la trichohyaline (O'Guin et al., 1992, J. Invest. Dermatol. 98: 24-32) et la kératine K6irs (Porter et al., 2001, Br.J.Dermatol. 145 :558-568) sont exprimées dans le follicule pileux en particulier dans la gaine interne mais pas dans l'épiderme, et que la cyclooxygénase de type 1, si elle est exprimée dans l'épiderme, ne l'est pas dans les kératinocytes du follicule pileux mais dans la papille dermique (Michelet. et al., 1997, J. Invest. Dermatol. 108: 205-209).

De manière surprenante, la demanderesse a maintenant mis en évidence qu'une enzyme spécifiquement impliquée dans la dégradation de ces prostaglandines est présente dans la papille dermique du cheveu, qui est un compartiment déterminant pour la vie du cheveu. En effet, la demanderesse a maintenant prouvé la présence de 15 hydroxy prostaglandine déshydrogénase (15 PGDH) à ce niveau. Elle a en outre montré que l'inhibition spécifique de la 15PGDH a un effet bénéfique sur la densité et/ou la croissance pilaire.

C'est pourquoi la présente invention a pour objet une composition, en particulier cosmétique, contenant au moins un inhibiteur de la 15-hydroxy prostaglandine déshydrogénase et des excipients physiologiquement acceptables, notamment sur un plan cosmétologique.
L'invention a ainsi pour objet l'utilisation cosmétique d'au moins un inhibiteur de 15-PGDH dans ou pour la préparation d'une composition destinée à augmenter la densité des fibres kératiniques et notamment des cheveux, et/ou réduire l'hétérogénéité de leur diamètre et/ou favoriser leur pousse.
L'invention a encore pour objet l'utilisation cosmétique d'au moins un inhibiteur de 15-PGDH dans une composition cosmétique de soin ou de maquillage des cils d'être humain ou pour la préparation d'une composition de soin et/ou de traitement des cils d'être humain, pour ou destinée à induire et/ou stimuler la croissance des cils et/ou augmenter leur densité. Cette composition permet ainsi de maintenir en bon état les cils et/ou d'améliorer leur aspect.

La 15-PGDH est une enzyme clé dans la désactivation des prostaglandines, en particulier de la PGF2α, et de la PGE2 , qui sont des médiateurs importants de la croissance et la survie du cheveu . La 15-PGDH de type 1 répond à la classification EC 1.1.1.141 et est NAD+ dépendante. Elle a été isolée de rein de porc; on a notamment observé son inhibition par une hormone thyroïdienne, la tri-iodo thyronine, à des doses très supérieures aux doses physiologiques. La 15-PGDH de type 2 est NADP dépendante.
Dans ce qui suit, l'expression 15-PGDH pourra désigner l'une ou l'autre forme de l'enzyme, ou les deux.
Cependant on n'avait jamais mis en évidence la présence de 15-PGDH dans la papille dermique, et il n'avait jamais été proposé d'utiliser un inhibiteur de 15PGDH pour maintenir et/ou augmenter la densité capillaire et/ou pour réduire l'hétérogénéité des diamètres de cheveux chez l'homme.

Par inhibiteur de la 15-PGDH on entend au sens de l'invention toute substance, composé simple ou complexe, d'origine naturelle ou synthétique, capable d'inhiber ou de diminuer l'activité de l'enzyme 15-PGDH, et/ou capable d'inhiber, diminuer ou ralentir la réaction catalysée par cette enzyme.
Avantageusement, l'inhibiteur est un inhibiteur spécifique de la 15-PGDH de type 1.

Par hétérogénéité des diamètres des cheveux on entend une grande variation des diamètres des cheveux sur une même zone du scalp ; certains cheveux ayant un diamètre physiologique voisin de 100 µm, d'autres ayant, au voisinage immédiat de ces cheveux, un diamètre diminué (cheveux fins). Ainsi par "réduire l'hétérogénéité des diamètres" , on entend augmenter le diamètre des cheveux fins.

Par augmenter la densité pilaire on entend augmenter le nombre de fibres kératiniques, cheveux ou cils par cm² de peau ou de cuir chevelu.

Avantageusement, une composition selon l'invention comprendra des excipients adaptés à une administration sur la zone cutanée ou la fibre kératinique visée, et notamment le cuir chevelu, les cheveux ou les cils. Le milieu dans lequel se trouvera l'inhibiteur de 15-PGDH selon l'invention peut être anhydre ou aqueux. La composition comprendra par exemple un milieu cosmétologiquement acceptable pouvant être constitué d'eau ou d'au moins un solvant choisi parmi les solvants organiques hydrophiles, les solvants organiques lipophiles, les solvants organiques amphiphiles et leurs mélanges, notamment un mélange d'eau et d'au moins l'un des solvants précités.

Pour une application topique, la composition utilisable selon l'invention peut être notamment sous la forme d'une solution aqueuse, hydroalcoolique ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H) ou multiples, d'une poudre libre ou compactée à utiliser telle quelle ou à incorporer dans un milieu physiologiquement acceptable, ou de suspensions ou émulsions de consistance molle du type crème ou gel aqueux ou anhydres, ou encore de microcapsules ou microparticules, ou de dispersions vésiculaires de type ionique et/ou non ionique. Elle peut ainsi se présenter sous forme d'onguent, de teinture, de crème, de pommade, de poudre, de timbre, de tampon imbibé, de solution, d'émulsion ou de dispersion vésiculaire, de lotion, de gel, de spray, de suspension, de shampooing, d'aérosol ou de mousse. Elle peut être anhydre ou aqueuse. Elle peut également consister en des préparations solides constituant des savons ou des pains de nettoyage.
Ces compositions sont préparées selon les méthodes usuelles.

La composition utilisable selon l'invention peut en particulier consister en une composition pour soins capillaires, et notamment un shampooing ou d'un après shampoing en particulier d'application bi-hebdomadaire ou hebdomadaire, une lotion de mise en plis, une lotion traitante, une lotion de soin capillaire par exemple d'application journalière ou bi-hebdomadaire, une crème ou un gel coiffant, des lotions restructurantes pour les cheveux, un masque, etc.

La composition cosmétique selon l'invention sera préférentiellement une crème, une lotion capillaire, un shampoing ou un après-shampoing, de mascara capillaire ou pour cils.

Pour une application sur les cils ou les poils, la composition à laquelle s'applique l'invention peut se présenter sous forme d'un mascara, pigmenté ou non, à appliquer à la brosse ou au peigne sur les cils, les sourcils, les cheveux, ou encore sur les poils de barbe ou de moustache.

Pour une composition à usage par injection, la composition peut se présenter sous forme de lotion aqueuse ou de suspension huileuse. Pour un usage par voie orale, la composition peut se présenter sous forme de capsules, de granulés, de sirops buvables ou de comprimés.

Les quantités des différents constituants des compositions utilisables selon l'invention sont celles classiquement utilisées dans les domaines considérés.

La phase aqueuse contient de l'eau et éventuellement un ingrédient miscible en toute proportion à l'eau comme les alcools inférieurs en C₁ à C₈ tel que l'éthanol, l'isopropanol, les polyols comme le propylène glycol, le glycérol, le sorbitol ou encore l'acétone ou l'éther.

Lorsque la composition utilisable selon l'invention est une émulsion, la proportion de la phase grasse peut aller de 2 à 80%, notamment de 5% à 80% en poids, et de préférence de 5% à 50% en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,1, notamment de 0,3% à 30% en poids, de préférence de 0,5 à 20% en poids, et mieux de 1 à 8% par rapport au poids total de la composition. L'émulsion peut, en outre, contenir des vésicules lipidiques et notamment des liposomes.

Lorsque la composition utilisable selon l'invention est une solution ou un gel huileux, la phase grasse peut représenter plus de 90% du poids total de la composition.

Avantageusement, la composition comprendra des microsphères, des nanosphères, des liposomes, des oléosomes ou des nanocapsules, dans lesquels au moins un agent inhibiteur de la 15-PGDH sera encapsulé. Des exemples de telles formulations sont décrits notamment dans les brevets EP199636, EP 375520, EP447318, EP557489, WO 97/12602, EP1151741 ou US 5 914126.

A titre d'exemple, les microsphères pourront être préparées selon la méthode décrite dans la demande de brevet EP 0 375 520.

Les nanosphères pourront se présenter sous forme de suspension aqueuse et être préparées selon les méthodes décrites dans les demandes de brevet FR 0015686 et FR 0101438.

Les oléosomes consistent en une émulsion huile dans eau formée par des globules huileux pourvus d'un enrobage cristal liquide lamellaire dispersé dans une phase aqueuse (voir les demandes de brevet EP 0 641 557 et EP 0 705 593).

L'inhibiteur de 15PGDH pourra aussi être encapsulé dans des nanocapsules consistant en un enrobage lamellaire obtenu à partir d'un tensio-actif siliconé tel que décrit dans la demande de brevet EP 0 780 115; les nanocapsules pourront également être préparées à base de polyesters sulfonique hydrodispersibles selon par exemple la technique décrite dans la demande de brevet FR 0113337.

Avantageusement pour une application capillaire, la composition est une solution ou suspension aqueuse, alcoolique ou hydro-alcoolique et mieux une solution ou suspension eau/éthanol. La fraction alcoolique peut représenter de 5% à 99,9% et mieux de 8% à 80%.

Pour une application mascara, la composition est une dispersion de cire-dans-eau ou de cire dans huile, une huile gélifiée, un gel aqueux, pigmenté ou non.

De façon connue, la composition selon l'invention peut contenir également des adjuvants habituels dans le domaine cosmétique, tels que les gélifiants ou épaississants hydrophiles ou lipophiles, les additifs hydrophiles ou lipophiles, les conservateurs, les antioxydants, les solvants, les parfums, les charges, les filtres, les absorbeurs d'odeur les électrolytes, les neutralisants, les agents bloqueurs d'U.V. comme les filtres solaires, les polymères filmogènes, les actifs cosmétiques et pharmaceutiques à action bénéfique pour la peau ou les fibres kératiniques et les matières colorantes, solubles ou non dans le milieu. Les quantités de ces différents adjuvants sont celles classiquement utilisées dans le domaine cosmétique, et notamment de 0,01 à 50% du poids total de la composition, par exemple de 0,01% à 20%, notamment inférieure ou égale à 10% du poids total de la composition, et en particulier supérieure ou égale à 0,1%. Ces adjuvants, selon leur nature, peuvent être introduits dans la phase grasse, dans la phase aqueuse et/ou dans les sphérules, vésicules ou microsphères lipidiques, telles que les liposomes.
La phase grasse peut contenir des composés gras ou huileux, liquides à température ambiante (25°C) et pression atmosphérique (760 mm de Hg), généralement appelés huiles. Ces huiles peuvent être compatibles ou non entre elles et former une phase grasse liquide macroscopiquement homogène ou un système bi- ou triphasique.
La phase grasse peut, en plus des huiles, contenir des cires, des gommes, des polymères lipophiles, des produits "pâteux" ou visqueux contenant des parties solides et des parties liquides.

Comme huiles ou cires utilisables dans l'invention, on peut citer les huiles minérales (huile de vaseline, isoparaffine hydrogénée), les huiles végétales (fraction liquide du beurre de karité, huile de tournesol, de soja, de blé), les huiles animales (perhydrosqualène), les huiles de synthèse (huile de Purcellin, esters d'acides gras), les huiles ou cires siliconées (polydiméthylsiloxanes linéaires ou cycliques, cyclométhicone, phényltriméthicone) et les huiles fluorées (perfluoropolyéthers), les cires d'abeille, de candelila, carnauba ou paraffine. On peut ajouter à ces huiles et cires des alcools gras et des acides gras libres (acide stéarique, linoléique, linolénique).
Comme émulsionnants utilisables dans l'invention, on peut citer par exemple le stéarate ou laurate de glycérol, les stéarates ou oléates de sorbitol, les alkyl diméthiconecopolyol (avec alkyle ≥ 8) et leurs mélanges; le stéarate ou oléate de sorbitol polyoxyéthyléné, par exemple le polysorbate 60 et le mélange de PEG-6/PEG-32/Glycol Stéarate vendu sous la dénomination de Tefose® 63 par la société Gattefosse, le monostéarate ou monolaurate de polyéthylène glycol, les diméthiconecopolyols et leurs mélanges.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les Bentones®, les sels métalliques d'acides gras comme les stéarates d'aluminium et la silice hydrophobe, éthylcellulose, polyéthylène.

De préférence, le ou les agents inhibiteurs sont présents à une concentration supérieure ou égale à 10⁻³ %, notamment de 0,001% à 5% p/v par rapport à la composition, de manière encore préférée de 0,01 à 2%. Toutefois ces quantités seront adaptées par l'homme du métier selon le composé utilisé, pour obtenir une activité anti-chute optimale et une activité d'inhibition enzymatique équivalente à une inhibition pratiquement totale de l'enzyme 15PGDH, dans les conditions d'application de la composition, la concentration utilisée étant généralement supérieure ou égale à celle pour laquelle une inhibition de 100% de la 15-PGDH est observée in vitro. La concentration en agent inhibiteur de 15-PGDH sera notamment 50 à 500 fois supérieure à la concentration pour laquelle une inhibition de 100% de la 15-PGDH a été observée in vitro, par exemple on utilisera de concentrations d'environ 100 fois celle correspondant à une inhibition totale in vitro.

Des inhibiteurs de la 15PGDH adaptés pourront être déterminés par l'homme du métier; l'agent inhibiteur sera notamment choisi parmi le traxanox, ses sels et ses esters.

Selon un mode de réalisation particulièrement avantageux de l'invention, la composition comprendra au moins un inhibiteur spécifique de la 15 PGDH; par inhibiteur spécifique on entend un actif qui soit peu ou pas inhibiteur de la synthèse des prostaglandines, en particulier de la synthèse de PGF2α ou de PGE2. De préférence, l'inhibiteur de la 15PGDH ne sera peu ou pas inhibiteur de la prostaglandine synthase (PGF synthase).

En effet, dans le cadre de ses recherches, la demanderesse a maintenant trouvé, de façon inattendue, que la PGF synthase est également exprimée dans la papille dermique. Le maintien d'une quantité efficace de prostaglandines au site d'action résulte donc d'un équilibre biologique complexe entre la synthèse et la dégradation de ces molécules. L'apport exogène de composés inhibant le catabolisme sera donc moins efficace si cette activité est combinée à une inhibition de la synthèse.
En complément ou remplacement de cet apport exogène, la demanderesse a maintenant mis en évidence qu'il est possible de favoriser le maintien d'un pool endogène de prostaglandines, et donc le maintien voire l'augmentation de la densité des fibre kératiniques, notamment de la densité capillaire, ainsi que le maintien de la qualité des fibres kératiniques, en particulier de cheveux.

En application de la présente invention, il est maintenant possible de cibler des composés particulièrement actifs, pour lesquels l'activité inhibitrice de la 15-PGDH est significativement supérieure à l'activité d'inhibition de la PGF synthase. Le rapport entre les activités inhibitrices respectivement de la 15PGDH et de la PGF synthase pour la dose administrée, déterminées notamment par les concentrations inhibitrices de 50% de l'activité enzymatique, sera au moins supérieur à 1, de préférence d'au moins 3:1, avantageusement supérieur ou égal à 5:1. Des agents particulièrement adaptés à la mise en oeuvre de l'invention présentent un ratio entre les activités inhibitrices de la 15PGDH et de la PGF synthase supérieur ou égal à 10:1, en particulier supérieur ou égal à 15, de préférence supérieur ou égal à 25 :1.
A cet égard, des composés adaptés à la mise en oeuvre de l'invention sont les composés répondant aux formules suivantes:
Molécule A: 5-amino-4,6-dichloro-2-phenyl-pyrimidine 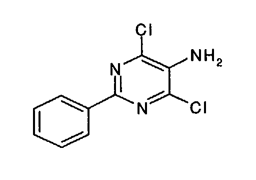
Molécule B: N-[7-(2-Chloro-phenyl)-5-oxo-5,6,7,8-tetrahydro-quinazolin-2-yl]-benzamide

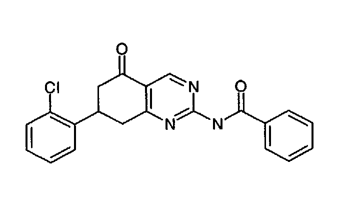

Le choix d'autres agents actifs sur l'inhibition de la 15-PGDH selon la présente invention pourra être effectué par l'homme du métier en mettant en oeuvre un test simple à partir de candidats potentiels. Ce test consistera à comparer la cinétique d'une réaction catalysée par cette enzyme, dans un milieu réactionnel comprenant un substrat de l'enzyme et d'éventuels co-substrats, en présence ou non du composé dont on veut évaluer le rôle d'inhibiteur de la 15-PGDH; les conditions de la réaction (pH, température, temps de réaction, etc,) sont celles adaptées à la réaction et sont les mêmes pour la mesure en présence ou en absence du composé ou de la substance à tester.
Pour cela, par exemple, on met en présence l'enzyme 15-PGDH à une concentration finale de 7. 10⁻³ mg/ml, son co-substrat (β-NAD) et un substrat (PGE2) aux concentrations correspondant aux conditions classiquement mises en oeuvre pour ce test tel que décrit par exemple par Cho et Taï, (Inhibition of NAD-dependent 15-hydroxyprostaglandin dehydrogenase (15-PGDH) by cyclooxygenase inhibitors and chemopreventive agents. Prostaglandins, Leukotrienes and Essential Fatty Acids, 2002 , 67(6) : 461-465) , par exemple 1,5 mM de β-NAD et 50µM de prostaglandine E2. On mesure la vitesse de réaction à 37°C pendant 1 minute. On réalise la même réaction, mais en ajoutant dans le milieu en début de réaction le composé à tester. On compare la vitesse maximale de réaction enzymatique par unité de temps (Vmax) mesurée en présence du composé à celle du témoin sans composé, et l'on détermine le pourcentage d'inhibition [100-(Vmax essai X 100) / Vmax témoin].
Les composés notés inhibiteurs de la 15PGDH sont alors testés pour leur capacité à inhiber la PGF synthase. Pour cela, par exemple, on met en présence l'enzyme PGFS à une concentration finale de 25. 10⁻³ mg/ml, son co-substrat (le β-NADPH2) et un substrat (par exemple la phénanthrène quinone) aux concentrations classiquement mises en oeuvre pour ce test tel que décrit par exemple par Suzuki et al. ( cDNA cloning, expression and mutagenesis study of liver-type prostaglandin F synthase. *J Biol Chem*, 1999, 274(1) : 241-248), c'est à dire 100 µM de β-NADPH2 et 20 µM de phénanthrène quinone. On mesure la vitesse maximale de réaction par unité de temps à 37°C. On réalise la même réaction, mais en ajoutant dans le milieu en début de réaction le composé à tester. On compare la vitesse maximale de réaction enzymatique avec le composé à celle du témoin sans composé, et l'on détermine le pourcentage d'inhibition [100-(Vmax essai X 100) / Vmax témoin].

On compare ensuite le pourcentage d'inhibition de la réaction catalysée par la 15-PGDH et celui de la réaction catalysée par la PGFS. De façon plus précise, on établit le ratio des IC50 d'un composé vis-à-vis de la PGFS et de la 15-PGDH (IC50 PGFS /IC50 15-PGDH). L'IC50 est la concentration du composé pour laquelle la Vmax est réduite de 50%.

L'activité de composés montrant une inhibition sélective de la 15-PGDH au sens de la présente invention pourra également être mise en évidence en mesurant le taux de prostaglandines dans un modèle cellulaire mimant l'environnement enzymatique de la papille du cheveu. Cela permet d'évaluer l'efficacité d'un inhibiteur sélectif de 15-PGDH sur la protection des prostaglandines dans un système biologique complexe produisant les différents types d'enzymes impliquées dans le métabolisme de ces molécules. Par exemple, on utilise une culture de promonocytes, qui sont des précurseurs des macrophages dans certaines conditions, modèle très répandu pour étudier le métabolisme des prostaglandines.
En effet, les esters de phorbol (PMA 10 nM) provoquent en 24h00 la différenciation de la lignée de promonocytes U937 en macrophages; cette différenciation s'accompagne par l'induction de la 15-PGDH. (Tong et Tai, *Biochim Biophys Acta*, 2000; 1497 : 61-68).
Par ailleurs, une stimulation de ces macrophages par du LPS (lipopolysaccharide extrait de paroi bactérienne) induit (à 100 ng/ml) en 6h00 la PGHS-2 (ou COX-2), enzyme responsable (au même titre que COX-1) de la synthèse de PGH2, précurseur (entre autres) via la PGFS ,de PGF2α. (Arias-Negrete et al., 1995. *Biochem Biophys Res Commun*, 208(2), 582-589).
Dans une 1^{ère} étape, on cultive des précurseurs de macrophages dans un milieu adapté, en présence d'un composé stimulant leur différenciation et l'induction de la 15-PGDH, puis on stimule la production de prostaglandines par ces cellules, par exemple par des LPS sous forme d'extrait ou purifiés; cette 2^{nde} étape est effectuée en présence ou non du composé à tester. On compare les concentrations de prostaglandines, en particulier de PGF2α obtenues en présence du composé inhibiteur de 15-PGDH à tester, à celle du témoin ne contenant que l'inducteur de 15-PGDH, cette mesure pouvant s'effectuer par toute méthode connue de l'homme du métier, notamment par dosage immuno-enzymatique. Au moment du dosage la quantité de PGF2α mesurée est donc la résultante des deux activités enzymatiques pour lesquelles les composés testés sont plus ou moins actifs : celle de la PGFS qui conduit à la synthèse de PGF2α et celle de la 15-PGDH qui conduit à la dégradation. En présence d'un inhibiteur non sélectif de la 15-PGDH (inhibiteur aussi de PGFS) on assistera à une baisse de PGF2α (correspondant à une baisse de la synthèse par action du produit sur la PGFS). En présence d'un inhibiteur sélectif de la 15-PGDH on assistera à une augmentation de PGF2α qui correspond à la baisse de la dégradation. Les composés pour lesquels le taux de PGF2α observé sont supérieurs d'au moins 5%, de préférence d'au moins 10% à celle du témoin (inducteur de la synthèse de prostaglandine seul) ont donc un rôle protecteur de la prostaglandine F2α. Avantageusement, pour les composés adaptés à la mise en oeuvre de l'invention la concentration de prostaglandines avec le composé à tester est supérieure ou égale d'au moins 20%, voire même de 30% à celle du témoin.

De tels composés sont notamment certains acétyl-tétrazoles, salifiés ou non, dotés d'une activité surprenante favorable à l'amélioration de la densité capillaire. La demanderesse a en effet trouvé que ces composés sont des inhibiteurs de la 15-hydroxy prostaglandine déshydrogénase.

Un autre objet de l'invention est donc une composition de soin capillaire contenant dans un milieu physiologiquement acceptable une quantité efficace d'un composé tétrazolique de formule (I) ou (II) ou d'un de ses sels : 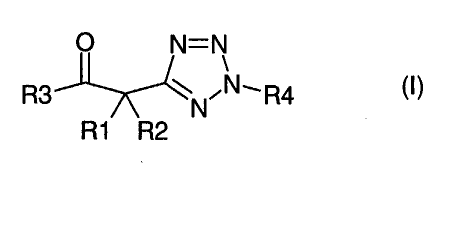 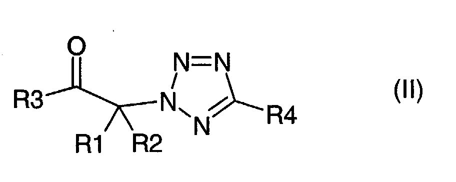 dans lesquelles :
- R₁ et R₂ sont indépendamment choisis parmi l'hydrogène, les halogènes, OR₅, SR₅, NR₅R'₅, COOR₅, COR₅, CONR₅R'₅, CF₃, CN, NR₅COR'₅, SO₂R₅, SO₂NR₅R'₅, NR₅SO₂R'₅, CSR₅, OCOR₅, COSR₅, SCOR₅, CSNR₅R'₅, NR₅CONR'₅R"₅, NR₅C(=NR'₅)NR"₅R"'₅, NR₅CSR'₅, NR₅CSNR'₅R"₅, les radicaux alkyle en C₁-C₂₀, linéaires ou ramifiés, et les cycles de 4 à 7 atomes, contenant éventuellement au moins un hétéroatome, ces cycles pouvant être séparés ou accolés, les radicaux alkyle et les cycles étant, en outre, saturés ou non, et éventuellement substitués par au moins un substituant A₁, où R₅, R'₅, R"₅ et R"₅ désignent indépendamment l'hydrogène, un radical alkyle en C₁-C₂₀, linéaire ou ramifié, ou un cycle hydrocarboné de 4 à 7 atomes, le cycle hydrocarboné ou le radical alkyle étant saturé ou non et éventuellement substitué par au moins un substituant A₂ ;
- R₃ est choisi parmi l'hydrogène, OR₆, SR₆, NR₆R'₆, CF₃, NR₆COR'₆, NR₆SO₂R'₆, NR₆CONR'₆R"₆, NR₆CSR'₆, NR₆CSNR'₆R"₆, les radicaux alkyle en C₁-C₂₀, linéaires
ou ramifiés, et les cycles hydrocarbonés de 4 à 7 atomes, séparés ou accolés, les radicaux alkyle et les cycles hydrocarbonés étant, en outre, saturés ou non et éventuellement substitués par au moins un substituant A₃, avec R₆, R'₆ et R"₆ désignant indépendamment l'hydrogène, un radical alkyle en C₁-C₂₀, linéaire ou ramifié, ou un cycle hydrocarboné de 4 à 7 atomes, le radical alkyle ou le cycle hydrocarboné étant saturé ou non et éventuellement substitué par au moins un substituant A₄ ;
- R₄ est choisi parmi l'hydrogène, COOR₇, CONR₇R'₇, SO₂R₇, SO₂NR₇R'₇, COR₇, CSR₇, COSR₇, CSNR₇R'₇, les radicaux alkyle en C₁-C₂₀, linéaires ou ramifiés, et les cycles hydrocarbonés de 4 à 7 atomes, séparés ou accolés, les radicaux alkyle et les cycles hydrocarbonés étant, en outre, saturés ou non et éventuellement substitués par au moins un substituant A₅ ; R₄ peut, en outre, représenter, dans le cas de la formule (II), un halogène, OR₇, SR₇, NR₇R'₇, CF₃, CN, NR₇COR'₇, NR₇SO₂R'₇, OCOR₇, SCOR₇, NR₇CONR'₇R"₇, NR₇C(=NR'₇)NR"₇R"'₇, NR₇CSR'₇ ou NR₇CSNR'₇R"₇, avec R₇, R'₇, R"₇ et R"'₇ désignant indépendamment l'hydrogène, un radical alkyle en C₁-C₂₀, linéaire ou ramifié, ou un cycle hydrocarboné de 4 à 7 atomes, le radical alkyle ou le cycle hydrocarboné étant saturé ou non et éventuellement substitué par au moins un substituant A₆ ;
- A₁ et A₂ sont indépendamment choisis parmi les halogènes, les hétérocycles comportant de 4 à 7 atomes et au moins un hétéroatome, OR₈, SR₈, NR₈R'₈, COOR₈, CONR₈R'₈, CF₃, CN, NR₈COR'₈, SO₂R₈, SO₂NR₈R'₈, NR₈SO₂R'₈, COR₈, CSR₈, OCOR₈, COSR₈, SCOR₈, CSNR₈R'₈, NR₈CONR'₈R"₈, NR₈C(=NR'₈)NR"₈R"'₈, NR₈CSR'₈, NR₈CSNR'₈R"₈ ;
- A₃ et A₄ sont indépendamment choisis parmi les halogènes, R₉, OR₉, SR₉, NR₉R'₉, COOR₉, CONR₉R'₉, CF₃, CN, NR₉COR'₉, SO₂R₉, SO₂NR₉R'₉, NR₉SO₂R'₉, CSR₉, OCOR₉, COSR₉, SCOR₉, CSNR₉R'₉, NR₉CONR'₉R"₉, NR₉C(=NR'₉)NR"₉R'''₉, NR₉CSR'₉, NR₉CSNR'₉R"₉ ;
- A₅ et A₆ sont indépendamment choisis parmi les halogènes, R₁₀, OR₁₀, SR₁₀, NR₁₀R'₁₀, CF₃, CN, NR₁₀COR'₁₀, SO₂R₁₀, SO₂NR₁₀R'₁₀, NR₁₀SO₂R'₁₀, CSR₁₀, OCOR₁₀, SCOR₁₀, CSNR₁₀R'₁₀, NR₁₀CONR'₁₀R"₁₀, NR₁₀C(=NR'₁₀)NR"₁₀R"'₁₀, NR₁₀CSR'₁₀, NR₁₀CSNR'₁₀R"₁₀ ;
- R₈, R'₈, R"₈, R'"₈, R₉, R'₉, R"₉, R"'₉, R₁₀, R'₁₀, R"₁₀ et R"'₁₀ désignant indépendamment l'hydrogène, un radical alkyle en C₁-C₂₀, linéaire ou ramifié, saturé ou non, un cycle hydrocarboné de 4 à 7 atomes, saturé ou non, ou un radical benzyle.

L'invention se rapporte encore à l'utilisation d'au moins un composé tétrazolique de formule (I) ou (II) ou forme tautomère ou de l'un de ses sels, tel que défini précédemment, comme agent pour induire et/ou stimuler la croissance des cheveux des êtres humains et/ou freiner leur chute et/ou augmenter leur densité.
L'invention se rapporte encore à l'utilisation cosmétique d'au moins un composé tétrazolique de formule (I) ou (II) ou de l'un de ses sels dans une composition cosmétique de soin capillaire d'être humain pour réduire la chute des cheveux et/ou augmenter leur densité. Elle a encore pour objet l'utilisation d'au moins un composé tétrazolique de formule (I) ou de l'un de ses sels pour la préparation d'une composition capillaire pour être humain, destinée à induire et/ou stimuler la croissance des cheveux et/ou freiner leur chute et/ou augmenter leur densité.

En particulier, l'invention se rapporte encore à l'utilisation cosmétique d'au moins un composé tétrazolique de formule (I) ou (II) ou de l'un de ses sels dans une composition cosmétique de soin capillaire d'être humain ou pour la préparation d'une composition capillaire d'être humain destinée à/ou pour traiter l'alopécie androgénique. Ainsi, cette composition permet de maintenir en bon état la chevelure et/ou lutter contre la chute naturelle des cheveux des hommes.

L'invention a encore pour objet l'utilisation d'au moins un composé tétrazolique de formule (I) ou (II) ou de l'un de ses sels comme inhibiteur de la 15-hydroxy prostaglandine déshydrogénase. Elle a encore pour objet l'utilisation d'au moins un composé tétrazolique de formule (I) ou (II) ou de l'un de ses sels pour la fabrication d'une composition destinée à traiter les désordres liés à la 15-hydroxy prostaglandine déshydrogénase chez l'être humain.
Avantageusement, les composés de formule (I) ou (II), sous forme salifiée ou non, présentent une activité inhibitrice de la 15-PGDH qui est supérieure à l'activité d'inhibition de la PGF synthase.
Dans la suite du texte, et sauf mention exprès, l'emploi du terme composé de formule (I) ou (II) doit être compris comme signifiant aussi bien le composé de formule (I) ou de formule (II), sous forme acide ou basique que sous forme de sel.

"Au moins un" selon l'invention signifie un ou plusieurs (2, 3 ou plus). En particulier, la composition peut contenir un ou plusieurs composés de formule (I), un ou plusieurs composés de formule (II) ou un mélange de composés de formule (I) et de formule (II). Ce ou ces composés peuvent être des isomères cis ou trans ou un mélange d'isomères cis/trans. Ils peuvent aussi être sous forme tautomère. Ils peuvent être également des énantiomères et/ou des diastéréoisomères ou un mélange de ces isomères, en particulier un mélange racémique.

Par cycle "hydrocarboné", on entend au sens de l'invention un cycle ne contenant que des liaisons carbone-carbone pour former le cycle.
Selon l'invention, les cycles employés pour R₁ à R₄ dans les formules (I) et (II) comportent indépendamment de 4 à 7 atomes et mieux de 5 à 6 atomes. Ils peuvent être saturés ou insaturés. Ils peuvent, de plus, être seuls ou accolés à un autre cycle de même structure chimique ou non. En outre, R₁ et R₂ comportent éventuellement un
ou plusieurs hétéroatomes tels que S, N, O ou leurs associations.

Selon l'invention, les cycles employés pour R₅, R'₅, R"₅, R"'₅, R₆, R'₆, R"₆, R"'₆, R₇, R'₇, R"₇, R"'₇, R₈, R'₈, R"₈, R"'₈, R₉, R'₉, R"₉, R"'₉, R₁₀, R'₁₀, R"₁₀ et R"'₁₀ dans les formules (I) et (II) comportent indépendamment de 4 à 7 atomes de carbone et mieux de 5 à 6 atomes de carbone. Ils peuvent être saturés ou mieux insaturés.

Par ailleurs, les hétérocycles employés pour A₁ et A₂ dans les formules (I) et (II) comportent un ou plusieurs hétéroatomes tels que S, N, O ou leurs associations. Ils comportent, en outre, indépendamment de 4 à 7 atomes et mieux de 5 à 6 atomes. De plus, ils peuvent être saturés ou insaturés.

Comme cycles hydrocarbonés saturés utilisables dans les formules (I) ou (II) on peut citer le radical cyclopentyle ou cyclohexyle. Comme hétérocycle, on peut citer les cycles pyridine, pipéridine, morpholine, pyrrole, furanne, thiazole. Comme cycles hydrocarbonés insaturés, on peut citer le radical phényle, naphtyle. En outre, ces cycles peuvent être substitués par un ou plusieurs substituants ayant la définition indiquée ci-dessus pour A₁, A₂, A₃, A₄, A₅ et A₆, selon qu'il s'agit de R₁, R₂, R₃, R₄, R₅, R'₅, R"₅, R"'₅, R₆, R'₆, R"₆, R₇, R'₇, R"₇ ou de R"'₇.

Par radical alkyle linéaire ou ramifié en C1-C20 on entend selon l'invention les radicaux acycliques provenant de l'enlèvement d'un atome d'hydrogène dans la molécule d'un hydrocarbure, linéaire ou ramifié ayant de 1 à 20 atomes de carbone et en particulier les radicaux méthyle, éthyle, propyle, isopropyle, butyle, isobutyle, tertiobutyle, pentyle, hexyle, heptyle, octyle ainsi que leurs isomères de position correspondant. Comme exemple de radical alkyle (saturé) utilisable dans l'invention, on peut citer les radicaux méthyle, éthyle, n-butyle, iso-propyle, n-hexyle.

Comme atome d'halogène, on peut utiliser les atomes de chlore, de fluor, d'iode ou de brome, et plus spécialement de chlore.

Selon l'invention, les composés de formule (I) ou (II) sont sous forme isolée, c'est-à-dire non polymérique. En outre, les substituants A₁, A₂, A₃, A₄, A₅ et A₆ peuvent être situés en toute position du cycle le portant et en particulier en position adjacente au groupement portant le cycle tétrazole.

Selon un mode de réalisation, l'un au moins des R₁ et R₂ représentent un atome d'hydrogène, un atome d'halogène et notamment un atome de fluor ou de chlore. En particulier, R₁ et R₂ représentent l'hydrogène.

Avantageusement, R₃ représente NR₆R'₆ ou un radical aryle, et dans un mode de réalisation particulier un radical naphtyle ou phényle, éventuellement substitué par le substituant A₃. En particulier, A₃ représente OR₉.

Selon un mode de réalisation, R₆ représente l'hydrogène et R'₆ un radical aryle, en particulier phényle, éventuellement substitué par le groupe OR₉.

En particulier, R₉ représente un radical alkyle en C₁-C₂₀ et mieux en C₁-C₁₀, linéaire ou ramifié, saturé et par exemple le radical méthyle.

Selon un mode de réalisation de l'invention, R₄ représente un radical aryle et notamment un radical naphtyle ou phényle.

Par sels de composé de formule (I) ou (II), on entend selon l'invention, les sels organiques ou inorganiques d'un composé de formule (I) ou (II).

Comme sels inorganiques utilisables selon l'invention on peut citer les sels de sodium ou de potassium ainsi que les sels de zinc (Zn²⁺), de calcium (Ca²⁺), de cuivre (Cu²⁺), de fer (Fe²⁺), de strontium (Sr²⁺), de magnésium (Mg²⁺) et de manganèse (Mn²⁺) ; les hydroxydes et les carbonates.

Les sels organiques utilisables selon l'invention sont par exemple les sels de tri-éthanolamine, mono-éthanolamine, di-éthanolamine, hexadécylamine, N,N,N',N'-tétrakis-(hydroxy-propyl-2) éthylène diamine, tris-hydroxyméthyl aminométhane.

Des composés adaptés à la mise en oeuvre de l'invention sont en particulier les composés inhibiteurs de 15-PGDH de formule (I) ou (II) telles que définies ci-dessus, dans lesquelles:
- R₁ et R₂ sont indépendamment choisis parmi l'hydrogène, les halogènes, OR₅, SR₅, NR₅R'₅, COOR₅, CF₃, CN, les radicaux alkyle en C₁-C₂₀, linéaires ou ramifiés, et les cycles de 4 à 7 atomes, contenant éventuellement au moins un hétéroatome, ces cycles pouvant être séparés ou accolés, les radicaux alkyle et les cycles étant, en outre, saturés ou non, où R₅ et R'₅ désignent indépendamment l'hydrogène, un radical alkyle en C₁-C₂₀, linéaire ou ramifié, ou un cycle hydrocarboné de 4 à 7 atomes, le cycle hydrocarboné ou le radical alkyle étant saturé ou non ;
- R₃ est choisi parmi l'hydrogène, OR₆, SR₆, NR₆R'₆, CF₃, les radicaux alkyle en C₁-C₂₀, linéaires ou ramifiés, et les cycles hydrocarbonés de 4 à 7 atomes, séparés
ou accolés, les radicaux alkyle et les cycles hydrocarbonés étant, en outre, saturés ou non et éventuellement substitués par au moins un substituant A₃, avec R₆ et R'₆ désignant indépendamment l'hydrogène, un radical alkyle en C₁-C₂₀, linéaire ou ramifié, ou un cycle hydrocarboné de 4 à 7 atomes, le radical alkyle ou le cycle hydrocarboné étant saturé ou non et éventuellement substitué par au moins un substituant A₄ ;
- R₄ est choisi parmi l'hydrogène, COOR₇, CSR₇, les radicaux alkyle en C₁-C₂₀, linéaires ou ramifiés, et les cycles hydrocarbonés de 4 à 7 atomes, séparés ou accolés, les radicaux alkyle et les cycles hydrocarbonés étant, en outre, saturés ou non ; R₄ peut, en outre, représenter, dans le cas de la formule (II), un halogène, OR₇, SR₇, NR₇R'₇, CF₃, CN , avec R₇ et R'₇, désignant indépendamment l'hydrogène, un radical alkyle en C₁-C₂₀, linéaire ou ramifié, ou un cycle hydrocarboné de 4 à 7 atomes, le radical alkyle ou le cycle hydrocarboné étant saturé ou non, éventuellement substitué par au moins un substituant choisi parmi OR₁₀, SR₁₀, NR₁₀R'₁₀, CF₃, avec R10 et R'10 désignant un radical alkyle en C₁-C₂₀ linéaire ou ramifié, saturé ou non;
- A₃ et A₄ sont indépendamment choisis parmi les halogènes, R₉, OR₉, SR₉, NR₉R'₉, COOR₉, CF₃, avec R₉ et R'₉, désignant indépendamment l'hydrogène, un radical alkyle en C₁-C₂₀, linéaire ou ramifié, saturé ou non, un cycle hydrocarboné de 4 à 7 atomes, saturé ou non, ou un radical benzyle.

Selon un autre mode de réalisation de l'invention, les composés inhibiteurs de la 15-PGDH sont tels que dans la formule (I) ou (II) définie précédemment,
- R₃ est choisi parmi l'hydrogène, OR₆, SR₆, NR₆R'₆, CF₃, les radicaux alkyle en C₁-C₂₀, linéaires ou ramifiés, et les cycles hydrocarbonés de 4 à 7 atomes, séparés ou accolés, les radicaux alkyle et les cycles hydrocarbonés étant, en outre, saturés ou non et éventuellement substitués par au moins un substituant A₃, avec R₆ et R'₆ désignant indépendamment l'hydrogène, un radical alkyle en C₁-C₂₀, linéaire ou ramifié, ou un cycle hydrocarboné de 4 à 7 atomes, le radical alkyle ou le cycle hydrocarboné étant saturé ou non et éventuellement substitué par au moins un substituant A₄ ;
   - R₄ est choisi parmi l'hydrogène, les radicaux alkyle en C₁-C₂₀, linéaires ou ramifiés, et les cycles hydrocarbonés de 4 à 7 atomes, séparés ou accolés, les radicaux alkyle et les cycles hydrocarbonés étant, en outre, saturés ou non éventuellement substitué par au moins un substituant choisi parmi OR₁₀, SR₁₀, NR₁₀R'₁₀, CF₃, avec R10 et R'10 désignant un radical alkyle en C₁-C₂₀ linéaire ou ramifié, saturé ou non;
   - A₃ et A₄ sont indépendamment choisis parmi R₉, OR₉, SR₉, NR₉R'₉, COOR₉, avec R₉ et R'₉, désignant indépendamment l'hydrogène, un radical alkyle en C₁-C₂₀, linéaire ou ramifié, saturé ou non, un cycle hydrocarboné de 4 à 7 atomes, saturé ou non, ou un radical benzyle.

Comme exemples de composés tétrazoliques de formule (I) utilisables dans l'invention on peut citer les composés suivants :

### Composé 1 : 1-phenyl-2-(2-phenyl-2H-tetrazol-5-yl)ethanone

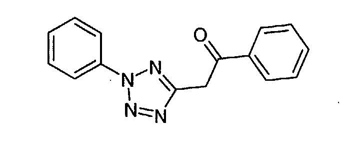

### Composé 2 : 1-(2-methoxyphenyl)-2-(2-phenyl-2H-tetrazol-5-yl)ethanone

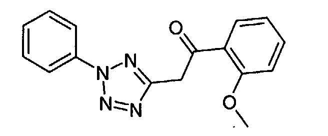

### ethyl-2-(2-phenyl-2H-tetrazol-5-yl)acetate

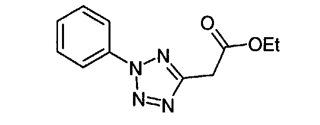

### acide 2-(2-phenyl-2H-tetrazol-5-yl)acétique

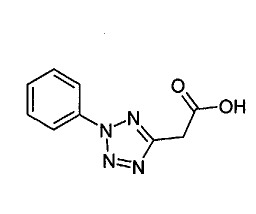

### 1-(3,4,5-trimethoxyphenyl)-2-(2-phenyl-2H-tetrazol-5-yl)ethanone

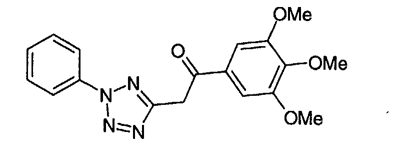

### 4-[2-(2-Phenyl-2H-tetrazol-5-yl)-acetyl]-benzoic acid

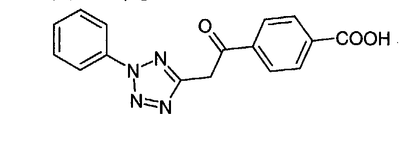

### 1-(4-Benzyloxy-phenyl)-2-(2-phenyl-2H-tetrazol-5-yl)-ethanone

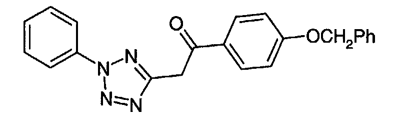

### 3-Methoxy-1-phenyl-2-(2-phenyl-2H-tetrazol-5-yl)-propan-1-one

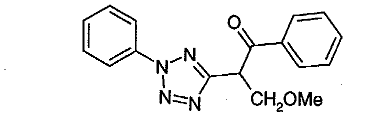

Comme exemples de composés tétrazoliques de formule (II) utilisables dans l'invention on peut citer les composés suivants :

### Composé 3 : N-(2-phenyl)-2-(5-phenyl-2H-tetrazol-2-yl)acetamide

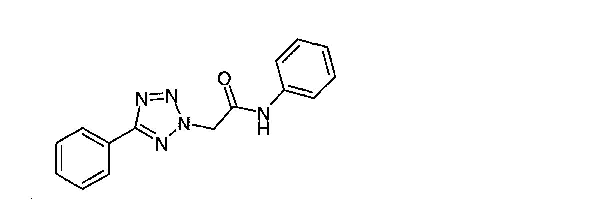

### Composé 4 : N-(2-methoxyphenyl)-2-(5-phenyl-2H-tetrazol-2-yl)acetamide

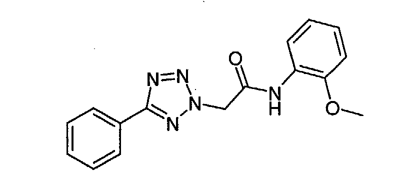

### N-(4-methylphenyl)-2-{5-[3-(trifluoromethyl)phenyl]-2H-tetrazol-2-yl}acetamide:

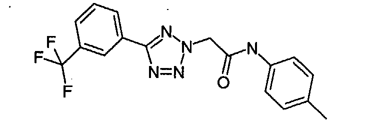

### N-(2-methoxyphenyl)-2-(2H-tetrazol-2-yl)acetamide

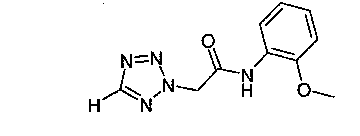

### 2-(5-phenyl-2H-tetrazol-2-yl)acetamide

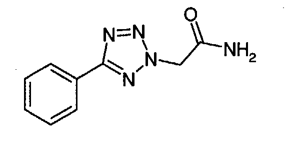

### N-(2-methoxyphenyl)-2-[5-(2-naphtyl)-2H-tetrazol-2-yl]acetamide

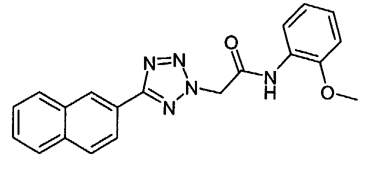

### N-(2-methoxyphenyl)-2-[5-(4-methoxyphenyl)-2H-tetrazol-2-yl)acetamide

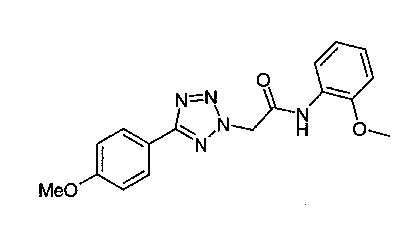

### N-(3-hydroxypropyl)-2-(5-phenyl-2H-tetrazol-2-yl)acetamide

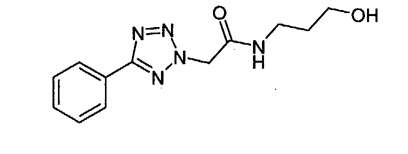

### 3-Hydroxy-N-(2-methoxy-phenyl)-2-(5-phenyl-tetrazol-2-yl)-propionamide

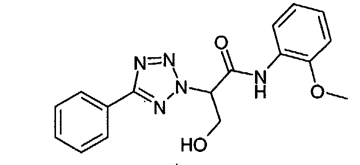

### 3-Methoxy-N-(2-methoxy-phenyl)-2-(5-phenyl-tetrazol-2-yl)-propionamide

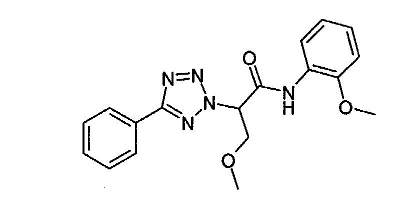

Les composés de formule (I) ou (II), salifiés ou non peuvent être fabriqués de façon connue.

### 1) Préparation de 5 - acétyl tétrazoles (formule I)

Les composés (I) de l'invention peuvent être préparés par une méthode décrite dans la littérature : D. Moderhack et al., J. Chem. Soc. Perkin Trans. 1, 2001, 720-728.
Le schéma réactionnel est le suivant:

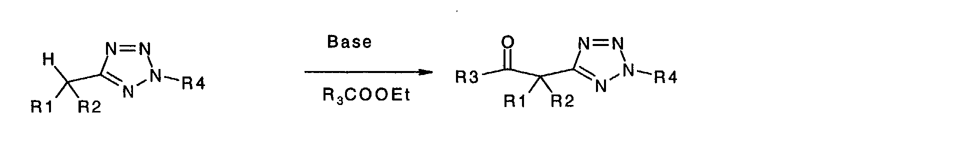

### 2)Préparation de 2 - acétyl tétrazoles (formule II)

Les composés de formule (II) de l'invention peuvent être préparés par l'alkylation par des réactifs α-chlorocarbonylés de tétrazoles substitués en position 5. Cette réaction est particulièrement adaptée dans le cas de la synthèse de 5-phényl-tétrazoles (correspondant à R₄ = phényle). Ce genre de préparation est connu de l'homme de l'art et notamment du document F. Eindberg, J. Org. Chem., 1970, 35, 11, 3978-3980.
Le schéma réactionnel peut être le suivant:

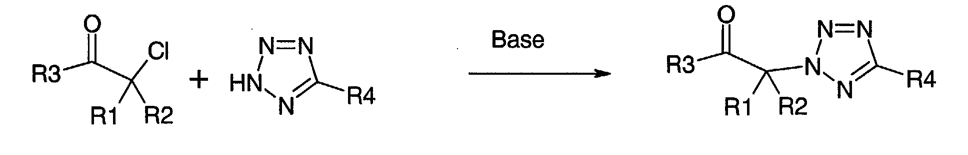

A la connaissance du demandeur, aucun document de l'art antérieur ne décrit ni ne suggère que les composés tétrazoliques de formule (I) ou (II) ou leurs sels aient la propriété d'induire et/ou de stimuler la croissance des cheveux et/ou de freiner leur chute ni que ces composés peuvent être utilisés par voie topique pour augmenter la densité des cheveux.

La quantité efficace d'un composé de formule (I) ou (II) ou de l'un de ses sels correspond à la quantité nécessaire pour obtenir le résultat désiré (à savoir augmenter la densité des cheveux). L'homme du métier est donc en mesure d'évaluer cette quantité efficace qui dépend de la nature du composé utilisé, de la personne à laquelle on l'applique, et du temps de cette application.

Dans la suite du texte, et sauf indication contraire, les quantités des différents ingrédients de la composition sont données en pourcentage en poids par rapport au poids total de la composition.

Pour donner un ordre de grandeur, selon l'invention, le composé de formule (I) ou de l'un de ses sels peut être utilisé en une quantité représentant de 10⁻³ % à 5% du poids total de la composition et préférentiellement en une quantité représentant de 10⁻² % à 2% du poids total de la composition, par exemple de 0,5 à 2 %.

La composition de l'invention peut être à usage cosmétique ou pharmaceutique. Préférentiellement, la composition de l'invention est à usage cosmétique. Aussi, la composition doit contenir un milieu physiologiquement acceptable non toxique et susceptible d'être appliqué sur la peau, les phanères ou les lèvres d'êtres humains. Par "cosmétique", on entend au sens de l'invention une composition d'aspect, d'odeur et de toucher agréables.

Le composé de formule (I) ou (II), salifié ou non, peut être utilisé dans une composition qui doit être ingérée, injectée ou appliquée sur la peau (sur toute zone cutanée à traiter).

Selon l'invention, le composé de formule (I) ou(I) peut être utilisé par la voie orale en une quantité de 0,1 à 300 mg par jour, par exemple de 5 à 10 mg/j.

Une composition préférée de l'invention est une composition à usage cosmétique et en particulier d'application topique sur la peau, et plus spécialement sur le cuir chevelu.

Selon un mode de réalisation avantageux, les compositions selon l'invention comprennent en outre au moins un agent bénéfique pour les cheveux tels que notamment les silicones, les huiles végétales, animales, minérales ou de synthèse, les cires, les céramides, les pseudocéramides, les polymères cationiques, les filtres solaires, les vitamines.
Les silicones utilisables conformément à l'invention sont en particulier des polyorganosiloxanes insolubles dans la composition et peuvent se présenter sous forme d'huiles, de cires, de résines ou de gommes.
Les organopolysiloxanes sont définis plus en détail dans l'ouvrage de Walter NOLL "Chemistry and Technology of Silicones" (1968) Academie Press. Elles peuvent être volatiles ou non volatiles.

Les agents inhibiteurs de 15PGDH seront, selon l'un des modes de réalisation de l'invention, associés à d'autres composés actifs pour favoriser la repousse et/ou limiter la chute des cheveux.

Ces composés pourront notamment être choisis parmi les inhibiteurs de lipoxygénase tels que décrits dans EP648488, les inhibiteurs de bradykinine décrits notamment dans EP 845700, les prostaglandines et leurs dérivés notamment ceux décrits dans WO 98/33497, WO 95/11003, JP 97-100091, JP 96134242, les agonistes ou antagonistes des récepteurs des prostaglandines, les analogues non prostanoïques de prostaglandines tels que décrits dans EP 1175891 et EP1175890, WO 01/74307, WO 01/74313, WO 01/74314, WO 01/74315 ou WO 01/72268.
Des agents favorisant la pousse du cheveux pouvant être présents dans les compositions selon l'invention incluent les vasodilatateurs, les antiandrogènes, les cyclosporines et leurs analogues, les anti-microbiens, les antifongiques, les anti-inflammatoires à l'exception des inhibiteurs sélectif de la prostaglandine H synthase 1 ou COX-1, les tri-terpènes, seuls ou en mélange.
Les vasodilatateurs tels que les agonistes des canaux potassium incluant le minoxidil et ses dérivés, l'aminexil et les composés décrits dans les brevets US 3 382247, 5 756092, 5 772990, 5 760043, 5 466694, 5 438058, 4 973474, la chromakalim et le diaxozide ou le nicorandil peuvent ainsi être présents dans les compositions.
Les anti-androgènes utilisables incluent notamment les inhibiteurs de 5α réductase, comme le finastéride et les composés décrits dans US 5 516779, l'acétate de cyprostérone, l'acide azélaïque, ses sels et ses dérivés et les composés décrits dans US 5 480913, le flutamide et les composés décrits dans les brevets US 5 411981, 5 565467 et 4 910226.
Les composés anti-microbiens peuvent être choisis parmi les dérivés du sélénium, le kétoconazole, l'octopirox, le triclocarban, le triclosan, le pyrithione zinc, l'itraconazole, l'acide asiatique, l'hinokitiol, mipirocine, et les composés décrits dans EP680745, le chlorhydrate de clinycine, le peroxyde de benzoyle ou de benzyl et la minocycline. Les anti-inflammatoires pourront être choisis parmi les inhibiteurs spécifiques de la Cox-2 comme par exemple le NS-398 et le DuP-697 (B.Batistini et al., DN&P 1994 ; 7(8) :501-511) et/ou les inhibiteurs des lipoxygénases, notamment de 5-lipoxygénase comme par exemple le zileuton (F.J.Alvarez & R.T.Slade, Pharmaceutical Res. 1992 ; 9(11) :1465-1473).
D'autres composés actifs pour favoriser la pousse et/ou limiter la chute des cheveux présents dans les compositions pourront également être choisi dans le groupe comprenant l'aminexil et ses dérivés, le 6-O-[(9Z, 12Z)-octadéc-9,12-diénoyl]hexapyranose tels que décrits dans FR027293, FR0212828, chlorure de benzlakonium, chlorure de benzethonium, phénol, oestradiol, maléate de chlorphéniramine, les dérivés de chlorophylline, cholestérol, cystéine, méthionine, nicotinate de benzyle, menthol, huile de menthe poivrée, panthoténate de calcium, panthénol, résorcinol, les inhibiteurs de la protéine kinase C, les activateurs de la prostaglandine H synthase 1 ou COX-1 tels que décrits dans FR2732597 les inhibiteurs de la glycosidase, les inhibiteurs de glycosaminoglycanase, les esters d'acide pyroglutamique, les acides hexosaccharidiques ou acyl-hexosaccharique, éthylènes aryl substitués, les amino acides N-acylés, les flavonoïdes, les dérivés et analoogues d'ascomycine, les antagonistes d'histamine, les triterpènes comme l'acide ursolique et les composés décrits dans US 5529769, US 5468888, US 5631282, les saponines, les inhibiteurs de protéoglycanase, les agonistes et antagonistes d'estrogènes, pseudotèrines, les cytokines et les promoteurs de facteurs de croissance, les inhibiteurs d'IL-1 ou d'IL-6, les promoteurs d'IL-10, les inhibiteurs de TNF, les vitamines, comme la vitamine D, les analogues de la vitamine B12 et le panthoténol, les hydroxyacides, benzophénones, les acides gras estérifiés tels que décrits dans FR027293, FR0212828 et l'hydantoïne.

Selon un mode de réalisation avantageux de l'invention, l'agent inhibiteur de 15 PGDH sera associé à un composé actif pour favoriser la pousse et/ou limiter la chute des cheveux susceptible d'être métabolisé par cette enzyme. En effet, grâce aux travaux de la demanderesse, on sait maintenant que certains composés classiquement utilisés pour favoriser la pousse et/ou retarder ou empêcher la chute des cheveux ont une activité moindre en raison de la présence de 15PGDH par diminution de la concentration et/ou disparition accélérée des substances du site d'action. Conformément à la présente invention, on peut désormais obtenir des compositions ayant une efficacité renforcée, en associant un composé actif pour favoriser la pousse et/ou retarder ou empêcher la chute des cheveux, susceptible d'être métabolisé par la 15PGDH et un inhibiteur de la 15PGDH tel que défini précédemment. On obtiendra ainsi une action synergique des actifs pour favoriser la pousse et/ou retarder ou empêcher la chute des cheveux dans ces compositions.

La composition contiendra par exemple en outre au moins un composé choisi parmi les prostaglandines, notamment la prostaglandine PGE1, PGE2, leurs sels, leurs esters, leurs analogues et leurs dérivés, notamment ceux décrits dans WO 98/33497, WO 95/11003, JP 97-100091, JP 96134242, en particulier les agonistes des récepteurs des prostaglandines . Elle peut notamment contenir au moins un composé tel les agonistes (sous forme acide ou sous forme d'un précurseur notamment sous forme ester) du récepteur de la prostaglandine F2 alpha (FP-R) à l'exemple du latanoprost, du fluprostenol, du cloprostenol,, le bimatoprost, l'unoprostone, les agonistes (et leurs précurseurs notamment les esters tels le travoprost) des récepteurs de la prostaglandine E2 (EP1-R, EP2-R, EP3-R, EP4-R) tel le 17 phényle PGE2, le viprostol, le butaprost, le misoprostol, le sulprostone, le 16,16 diméthyle PGE2, 11 désoxy-PGE1, le 1 désoxy PGE1, les agonistes et leurs précurseurs notamment les esters du récepteur de la prostacycline (IP) tel le cicaprost, l'iloprost, l'isocarbacycline, le beraprost, les agonistes et leurs précurseurs notamment les esters du récepteur de la prostaglandine D2 tel le BW245C ((4S)-(3-[(3R,S)-3-cyclohexyl-3-isopropyl]-2,5-dioxo)4-imidazolidine-heptanoic acid), le BW246C ((4R)-(3-[(3R,S)-3-cyclohexyl-3-isopropyl]-2,5-dioxo)4-imidazolidineheptanoic acid), les agonistes et leurs précurseurs notamment les esters du récepteur aux thromboxanes A2 (TP) tel le I-BOP ([1S-[1a,2a(Z), 3b(1E,3S),4a]]-7-[3-[3-hydroxy-4-[4-(iodophenoxy)-1-butenyl]-7-oxabicyclo[2.2.1]hept-2-yl]-5-heptenoic acid).

Avantageusement, la composition selon l'invention comprendra au moins un inhibiteur de la 15-PGDH tel que défini précédemment et au moins une prostaglandine ou un dérivé de prostaglandine comme par exemple les prostaglandines de la série 2 dont notamment PGF 2 α et PGE 2 sous forme salines ou sous forme de précurseurs, notamment des esters (exemple les isopropyl esters), leurs dérivés comme le 16,16 dimethyl PGE2, le 17 phényl PGE2, le 16,16 diméthyle PGF2α, le 17 phényl PGF2α les prostaglandines de la série 1 comme le 11 déoxy prostaglandine E1 , le 1 déoxy prostaglandine E1 sous forme salines ou esters, leurs analogues notamment le latanoprost, le travoprost, le fluprostenol, l'unoprostone, le bimatoprost, le cloprosténol, le viprostol, le butaprost, le misoprostol, leurs sels ou leurs esters.
De manière préférée, la composition contient au moins un agoniste du récepteur FP tel que décrit par exemple dans WO03/009820 ou un agoniste prostanoïque ou non prostanoïque des récepteurs EP2 et/ou EP4 notamment tel que décrit dans EP 1175892.

Les compositions selon l'invention pourront également contenir des agents accélérateurs de pénétration. Ces composés sont connus de l'homme du métier et améliorent le passage des agents vers le site d'action; ils seront classiquement présents dans les compositions à des concentrations supérieures ou égales à 0,01 %, notamment de 0,1 à 20% et de préférence de 0,1 à 5%. Des composés de ce type utilisable sont par exemple l'urée et les composés cités dans la demande WO01/74313.

Selon un autre de ses objets, l'invention concerne un procédé de traitement cosmétique pour favoriser la pousse et/ou diminuer, empêcher ou retarder la chute des cheveux, caractérisé en ce qu'on inhibe ou diminue l'activité et/ou l'expression de la 15-PGDH par le follicule pileux, comprenant l'administration d'au moins un inhibiteur de la 15-PGDH Ce procédé sera adapté en particulier pour maintenir ou augmenter la densité capillaire du cheveu et diminuer l'hétérogénéité du diamètre capillaire, dans les cas physiologiques où ces paramètres sont perturbés, de façon transitoire. L'administration du ou des agents inhibiteurs de la 15PGDH sera notamment effectuée sous forme d'une composition telle que définie précédemment.

L'administration de l'inhibiteur de 15PGDH ou de la composition le contenant dans le procédé selon l'invention pourra être effectuée par toute voie, notamment orale. Toutefois, le procédé est mis en oeuvre de préférence par administration topique, in situ.

Les inhibiteurs de la 15 PGDH utilisés dans le procédé selon l'invention sont tels que définis précédemment; en particulier, on utilisera au moins un inhibiteur de la 15-PGDH spécifique, c'est à dire peu ou pas inhibiteur de la PGFS.

Selon l'une des variantes du procédé selon l'invention, on administre en outre une prostaglandine et/ou un dérivé de prostaglandine, comme par exemple les prostaglandines de la série 2 dont notamment PGF 2 α et PGE 2 sous forme salines ou esters (exemple les isopropyl esters), leurs dérivés comme le 16,16 dimethyl PGE2, le 17 phényl PGE2, le 16,16 diméthyle PGF2α, le 17 phényl PGF2α les prostaglandines de la série 1 comme le 11 déoxy prostaglandine E1, le 1 déoxy prostaglandine E1 sous forme salines ou esters, leurs analogues notamment le latanoprost, le fluprostenol, l'unoprostone, le bimatoprost, le cloprosténol, le viprostol, le butaprost, le misoprostol sous forme salines ou esters, tel le travoprost.
Le ou les agents inhibiteurs de la 15PGDH pourront être appliqués seuls ou en mélange, et éventuellement en association avec d'autres agents actifs pour favoriser la pousse et/ou retarder ou empêcher la chute des cheveux, ou des accélérateurs de pénétration tels que définis précédemment.
On peut, par exemple, appliquer la composition contenant une quantité efficace d'un composé inhibiteur de 15-PGDH, par exemple de formule (I) ou (II), salifié ou non, le soir, garder celle-ci au contact toute la nuit et éventuellement effectuer un shampooing le matin. Ces applications peuvent être renouvelées quotidiennement pendant un ou plusieurs mois suivant les individus.

Ainsi, la présente invention a également pour objet un procédé de traitement cosmétique des cheveux et/ou du cuir chevelu, destiné à stimuler la croissance des cheveux d'être humain et/ou freiner leur chute, caractérisé par le fait qu'il consiste à appliquer sur les cheveux et/ou le cuir chevelu, une composition cosmétique comprenant une quantité efficace d'au moins un composé inhibiteur de 15-PGDH, par exemple de formule (I) ou (II) ou d'un de ses sels, à laisser celle-ci en contact avec les cheveux et/ou le cuir chevelu, et éventuellement à rincer les cheveux et/ou le cuir chevelu.

Ce procédé de traitement présente les caractéristiques d'un procédé cosmétique dans la mesure où il permet d'améliorer l'esthétique des cheveux en leur donnant une plus grande vigueur et un aspect amélioré. En outre, il peut être utilisé quotidiennement pendant plusieurs mois.

Avantageusement, dans le procédé selon l'invention, on administre entre 5 et 500 µL d'une solution ou composition telle que définie précédemment, comprenant entre 0,001% et 5 % d'inhibiteur.

Selon encore un autre de ses aspects, l'invention a pour objet l'utilisation d'au moins un inhibiteur de la 15PGDH tel que défini précédemment, pour la préparation d'une composition destinée à diminuer la chute des cheveux et/ou à favoriser la repousse de cheveux chez un mammifère, et en particulier chez l'homme. La dite composition contiendra en outre des excipients physiologiquement acceptables, adaptés à une administration par voie topique ou systémique. Les inhibiteurs de 15PGDH seront notamment utilisés selon l'invention pour la préparation de composition, en particulier pharmaceutiques, dermatologiques ou cosmétologiques, en association avec des excipients connus de l'homme du métier. Toutes les variantes des compositions cosmétologiques décrites dans ce qui précède pourront être adaptées à la préparation de ladite composition.
Selon l'un de ses modes de réalisation, les compositions de l'invention sont destinées à prévenir, diminuer ou ralentir la chute des cheveux, notamment au traitement de l'alopécie, en particulier de l'alopécie androgénétique; l'alopécie androgénétique est distincte des alopécies auto-immunes telle que alopecia areata.

Les exemples qui suivent sont destinés à illustrer l'invention sans aucunement en limiter la portée

Dans ces exemples on se référera aux figures suivantes:
Figure 1: expression de la 15-hydroxy PGDH dans les follicules pileux
Figure 2 : expression de la prostaglandine synthase dans les follicules pileux
Figure 3: analyse électrophorétique de la 15PGDH et de la PGFS purifiées

### Exemple 1: Mise en évidence de l'expression de l'ARNm de la 15PGDH et de PGFS dans les fibroblastes de papilles dermiques de cheveux en culture.

### 1. Dissection des follicules pileux.

Des follicules pileux provenant de lifting de donneurs volontaires sont disséqués selon la méthode décrite dans le brevet B.Bernard/O.Gaillard FR2736721A1 du 17/01/97; US5712169A du 28/01/98.
Les follicules isolés sont placés en immersion dans une boite de pétri contenant 20 ml de milieu de culture 199 Gibco référence 31153-018, Life technologie, BP 96, Cergy Pontoise Cedex, supplémenté à 1% (v/v) d'une solution d'antibiotiques Gibco référence 15240-096.
A l'aide de deux aiguilles, référence NN-2516R, Terumo europe N.V. Leuven, Belgique montées chacune sur une seringue de 1ml, référence BS-01N, Terumo, 15 papilles dermiques sont extraites des bulbes folliculaires.
Ces 15 papilles sont introduites dans une boite de pétri de 35 mm de diamètre contenant 2 ml de milieu 199 précédemment utilisé contenant 20 % de sérum de veau foetal Gibco, référence 10091-130. La boite est ensuite placée dans un incubateur thermostaté à 37°C sous 5% de CO2.

Un premier passage est réalisé après 3 semaines d'incubation sans que le milieu n'ait été préalablement remplacé. Le milieu est éliminé, 1 ml de solution de trypsine Gibco référence 25050-022 est introduit dans la boite de pétri, la boite est replacée dans l'incubateur 4 minutes. Les cellules (fibroblastes de papilles) ainsi remises en suspension dans la solution de trypsine (contrôle microscopique) sont introduites dans un tube de 15 ml, référence Falcon, 352097, Becton Dickinson, Chemin des sources, BP 37, Meylan 38241, contenant 10 ml de milieu 199 précédemment utilisé (contenant 20% de sérum de veau foetal).
Après centrifugation 5 min à 1500 tours par minute, le surnageant est éliminé et le culot cellulaire repris par 5 ml de milieu 199 précédemment utilisé (contenant ici 10% de sérum de veau foetal).
La suspension cellulaire est introduite dans une boite de pétri de 35 mm de diamètre et replacée dans l'incubateur (37°C, 5% de CO2).
Les passages suivant P2,P3,P4 sont réalisés selon le même principe. Ceux-ci sont effectués dès lors que les cellules ont atteint la confluence.

### 2. Extraction et purification des ARN messagers.

L'extraction des ARN messagers des fibroblastes de papilles dermiques (réalisée sur les passages P3 ou P4, boite de 35 mm à confluence) est effectuée selon le protocole et avec les réactifs du kit QuickPrepr mRNA (Pharmacia Biotech, Bruxelles, Belgique).
Pour chaque échantillon (une culture cellulaire à confluence dans une boite de 35 mm de diamètre) mis à l'étude, le protocole suivant sera appliqué.
Le surnageant de culture cellulaire est éliminé et remplacé par 800 µl de tampon de lyse, le lysat obtenu est récupéré et introduit dans un microtube en polypropylène de 1,5 ml (tube 1).
1 ml de suspension de microsphères Oligo(dT-18) cellulose est introduit dans un microtube de 1,5 ml (tube 2) et centrifugé à 14000 trs/min pendant 1 minute. Le surnageant est éliminé. Le contenu du tube 1 est alors introduit dans le tube 2, les microsphères sont remise en suspension dans le lysat par agitation douce du tube pendant 3 minutes.
Les ARNs polyA+ fixés sur les microsphères sont isolés des contaminants par des lavages. Le tube est centrifugé à 14000 trs/min pendant 1 minute, le surnageant est éliminé et remplacé par 1 ml de tampon de lavage (high salt buffer). Les microsphères sont remises en suspension comme précédemment et le tube est agité doucemment pendant 1 minute. Le tube est centrifugé à 14000 trs/min pendant 1 minute, le surnageant est à nouveau éliminé et remplacé par 1ml de low salt buffer.
Au total cinq lavages avec le low salt buffer suivis de trois lavages avec le high salt buffer seront ainsi effectués.
Le contenu du troisième lavage (microsphères + tampon) est introduit sur une microcolonne contenant un filtre à la base (colonne microspin™) placée dans un microtube de 1,5 ml. L'ensemble est centrifugé à 14000 trs/min pendant 1 minute. La microcolonne est récupérée et placée dans un microtube de 1,5 ml. Les ARN messagers polyA+ sont alors élués par un volume final total de 0,4 ml de tampon d'élution préalablement porté à 65°C.

### 3. Précipitation des ARN messagers.

Dans le tube contenant l'éluat sont introduits 10 µl de solution de glycogène, 40 µl d'acétate de potassium 2,5M et 1 ml d'éthanol absolu à -20°C. Le tube est placé dans de la carboglace (-80°C). Après 1h00, le tube est centrifugé à 4°C à 17500 trs/min pendant 15 minutes. Le surnageant est précautionneusement éliminé (les ARNms forment un tout petit culot) et remplacé par 1ml d'éthanol 80% (éthanol/eau ; v/v) à - 20°C. Le tube est centrifugé 15 min à 17500 trs/min à 4°C et le surnageant complétemment éliminé. Le culot est repris par 8 µl d'eau distillée stérile.

### 4. Synthèse des brins complémentaires d'ADN (ADNc)

Cette étape est réalisée par l'utilisation du kit First strand cDNA synthesis (Pharmacia Biotech, Bruxelles, Belgique).
Le tube contenant les ARNms sont placés à 65°C pendant 10 minutes puis dans la glace pendant 5 minutes, sont ensuite introduits :
5 µl d'une solution tamponnée contenant une suspension de transcriptase inverse.
1µl d'amorces OligodT(18) à 0,8 µg/ml.
1 µl de solution aqueuse de dithiothréitol titrant 200 nM.
Le tube est incubé à 37°C pendant 1h00. La réaction est bloquée en plaçant le tube dans la glace.

### 5. Choix des amorces, réaction de polymérisation en chaîne (PCR)

1µl (d'une dilution au 1/10 ème dans de l'eau distillée stérile) d'ADNs complémentaires ainsi obtenus est soumis dans un milieu tamponné à une réaction de polymérisation en chaîne (PCR), en présence de couples d'amorces spécifiques (titrant 40 ng/ml), de TAQ Polymérase et de nucléotides selon les données du fournisseur.

Les amorces spécifiques des séquences d'intérêts qui seront utilisées sont obtenues par synthèse à façon par Genset SA, rue Robert et Sonia Delaunay, Paris.
Le premier couples d'amorces s'hybridant sur la séquence codant pour une protéine ubiquitaire (la βactine). Le deuxième couple d'amorces s'hybridant sur la séquence codant pour la 15 hydroxyprostaglandine déshydrogénase.

### β Actine humaine; N° accession genbank: NM_001101

AMORCE SENS: 5'-ATGGATGATGATATCGCCGCGCT-3'

Amorce anti-sens: 5'-CGGACTCGTCATACTCCTGCTTG-3'

Fragment amplifié: 1096 paires de bases.

### 15 hydroxyprostaglandine humaine (15PGDH); N°accession genbank: NM_000860

Amorce sens: 5'-TGCCAATGGATTGATAACACTCAT-3'

Amorce anti-sens: 5'-ACAGCAGTTTTCATCTGGGATATG-3'

Fragment amplifié: 706 paires de bases.

### Prostaglandine F synthase (PGFS) ; N° accession genbank : AB018580

Amorce sens: 5'-AATTCCGGGCAGCAAACA T-3'

Amorce anti-sens: 5'-ACACACAGGGCTTCTGGTAGACA-3'

Fragment amplifié: 1061 paires de bases.

La réaction PCR est réalisée selon une adaptation du protocole TAKARA Taq™, TAKARA Shuzo Co., LTD. Biomedical Group, Seta 3-4-1, Otsu, Shiga, 520-2193, Japon. La température d'hybridation est de 54°C pour les couples d'amorces (β-actine ;PGFS, 15PGDH), le nombre de cycles =35.
*Une solution tamponnée de nucléotides est réalisée par mélange de 171 µl d'eau distillée stérile, 24,5 µl de tampon 10X du kit et 20 µl de mélange de nucléotides (dNTP) du kit.
Sont introduits dans un microtube adapté à la PCR :
1 µl (d'une dilution au 1/10) d'ADN complémentaire
43 µl du mélange de nucléotides en solution tamponnée *
5 µl (2,5 µl + 2,5 µl) des couples d'amorces à 40 ng/µl sont en réaction.
50 µl d'huile minérale.

Le tube est placé dans un appareil pour PCR et les cycles suivants sont programmés.

| | |
|---|---|
| 4' à 95°C | 1 cycle |
| 30" à 94°C | |
| 1' à 54°C | 35 cycles |
| 1' à 72°C | |
| 7' à 72°C | 1 cycle |

### 6. Lecture

### 6.1 Préparation d'un gel d'agarose.

Pesée de 0,65 g d'agar (Molecular biology certified agarose, Bio-Rad laboratories 2000 Alfred Nobel Dr., Hercules, CA 94547, USA).
Ajout de 50 ml de tampon 1X TAE, Amresco, Solon, OHIO 44139, USA.
L'agarose en suspension est porté à ébullition puis introduit dans une cuve contenant une goutte de bromure d'éthydium (25 µg), Amresco, Solon, OHIO 44139, USA.
Un "peigne" permettant le dépôt des échantillons est placé à une extrémité de la cuve. Après 30 minutes de refroidissement (température de la pièce), 20 µl des résultats de PCRs sont introduits individuellement dans un puits du gel, de même que 10 µl d'un mélange de standards de poids moléculaires (Amplisize ™, molecular Ruler, 170-8200, Bio-Rad laboratories 2000 Alfred Nobel Dr., Hercules, CA 94547, USA)..
L'ensemble est soumis en immersion dans un large excès de tampon TAE 1X à un champ électrique de 100 volts pendant 45 minutes.

L'exposition du gel sous un éclairage ultra-violet permet d'observer par fluorescence, les résultats obtenus.

Poids des amplimères attendus.
Actine = 1096 paires de bases; 15-PGDH = 706 paires de bases; PGFS = 1061 paires de bases.
Les échantillons 1,2,3 proviennent de cultures différentes de fibroblastes de papilles dermiques de cheveux humains.

### Recherche de l'expression de la 15 hydroxyprostaglandine déshydrogénase.

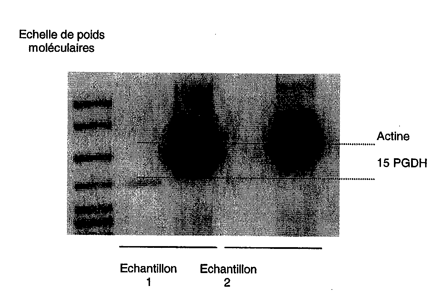

Les résultats sont présentés sur la figure 1
On constate que la 15-PGDH est exprimée dans les différents échantillons, avec une bande de PM caractéristique à 706 pb

### Recherche de l'expression de la prostaglandine F synthase.

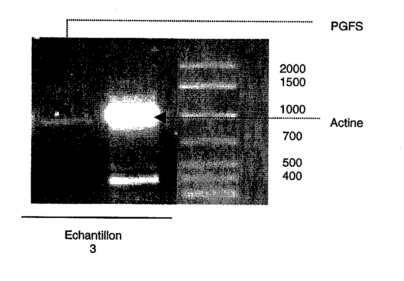

Les résultats sont présentés sur la figure 2
On constate que la PGFS est exprimée dans les différents échantillons, avec une bande de PM caractéristique 1061 pb

### Exemple 2.Clonage à partir de fibroblastes de papilles dermiques de cheveux puis purification de la PGFS et la 15 PGDH.

Ont été réalisées, à partir de culture de fibroblastes de papilles dermiques de cheveux tel que décrit dans l'exemple 1 l'extraction, la purification des ARN messagers poly-A+ puis la synthèse d'ADN complémentaire (ADNc).
Des couples d'amorces (synthétisé par Genset) auxquels ont été ajoutés des séquences codant pour des sites de restrictions ont été choisis pour la 15-PGDH (n° d'accession Genbank = NM_000860) et pour la PGFS (N°d'accession Genbank = AB018580).

### a) Amorces pour la 15-PGDH

5'-GGG GAT CCA TGC ACG TGA ACG GCA AAG TG-3' ; Amorce sens site
BamH1 (en gras)

5'-TCT CGA GAG CTG TTC ATT GGG T-3' ; Amorce anti-sens site Xho1 (en gras)

### b) Amorces pour la PGFS.

5'-CGG GAT CCA TGG ATT CCA AAC AGC AGT GTG-3' ; Amorce sens site
BamH1 (en gras)

5'-CG GAA TTC TTA ATA TTC ATC TGA A-3' ; Amorce anti-sens site EcoR1 (en
gras)

### c) Réaction de polymérisation en chaîne (PCR)

Le protocole de PCR appliqué pour le clonage de la 15-PGDH comme pour le clonage de la PGFS reprend dans les grandes lignes celui décrits précédemment à la différence près ci-dessous :

La Taq Polymérase utilisée selon les données du fabricant (Pfu Turbo^{r} DNA Polymerase), Stratagene cloning systems, 11011 North Torrey Pines Road , La Jolla, CA 92037.

Température d'hybridation 59°C, temps d 'élongation 2 min, 25 cycles au total pour la 15-PGDH, amplimère attendu = 815 pb.
Température d'hybridation 50°C, temps d'élongation 2 min, 25 cycles au total pour la PGFS, amplimère attendu 989 pb.
d) Les produits de PCR sont digérés par les enzymes de restrictions (BamH1 ; Xho1 pour la 15-PGDH et BamH1 ; EcoR1 pour la PGFS) selon les données du fabricant (Amersham Pharmacia biotech, 12 avenue des Tropiques, ZA Courtaboeuf, 91944 Les Ulis) puis mis à migrer individuellement sur un gel d'agarose à 1,3 % (voir exemple 1 ; 6. Lecture)
e) Découpe des bandes correspondantes aux amplimères attendus (voir ac) à l'aide d'un scalpel (après repérage sous ultra-violets) et purification de ces découpes selon les recommandations du fabricant du kit Wizard^{r} PCR Preps DNA Purification system (Promega Corporation, 2800 Woods Hollow Road, Madison, Wl 53711-5399).
f)
   15-PGDH
   Ligation dans un vecteur pGEX 4T3 (Amersham Pharmacia biotech, 12 avenue des Tropiques, ZA Courtaboeuf, 91944 Les Ulis) préalablement digéré (BamH1 / Xho1) et purifié, suivant les indications du fabricant du kit Fast-Link™ DNA ligation kit, Epicentre, 1202 Ann Street, Wl 53713.

### PGFS

Ligation dans un vecteur pGEX 2T (Amersham Pharmacia biotech, 12 avenue des Tropiques, ZA Courtaboeuf, 91944 Les Ulis) préalablement digéré (BamH1 / EcoR1) et purifié, suivant les indications du fabricant du kit Fast-Link™ DNA ligation kit, Epicentre, 1202 Ann Street, Wl 53713. Ces deux vecteurs permettent la synthèse de la protéine d'intérêt couplée à une protéine de fusion (la glutathion sulfo-transférase). Cette protéine de fusion permettra la purification ultérieure de la protéine d'intérêt.

### g) Transformations.

Des bactéries compétentes de type BL21DE3plys seront utilisées pour la transformation avec la construction (pGEX4T3/15-PGDH), des bactéries compétentes de type BL21DE3 pour la transformation avec la construction (pGEX 2T/PGFS). Ces deux souches sont commercialisées par la société Stratagene. Les transformations sont réalisées selon un protocole classiquement appliqué tel que décrit par exemple dans le kit Fast-Link™ DNA ligation précédemment utilisé. Les bactéries infectées (clones) sont sélectionnées (colonies blanches) après dépôt et culture 24h00 à 37°C d'une fraction de ces produits de transformations sur LB-Agar medium coulée en boite de pétri (L-2897, Sigma-Aldrich, L'isle D'Abeau Chesne, BP 701, 38297, Saint Quentin Fallavier) contenant 100 µg/ml d'ampicilline.

### h) Production de la 15-PGDH et de la PGFS.

Une colonie provenant de la boite de pétri « transformation 15-PGDH » est prélevée et introduite dans 250 ml de milieu LB (L-3022, Sigma-Aldrich, L'isle D'Abeau Chesne, BP 701, 38297, Saint Quentin Fallavier) contenat 100 µg/ml d'ampicilline, le flacon est incubé sous agitation 16h00 à 37°C.
Il est procédé de même dans un autre flacon, pour une colonie issue de la boite de pétri « transformation PGFS »
Après 16h00, chacun des flacons est introduit dans un erlen contenant 2,5 L de milieu LB contenant 100 µg/ml d'ampicilline. Ces deux erlens sont incubés sous agitation à 37°C pendant 3h00 à 4h00 (jusqu'à ce que la densité optique mesurée à 630 nm soit comprise entre 0,6-0,9.
Ajout d'isopropyl β-D thiogalactopyranoside (IPTG), (16758, Sigma-Aldrich, L'isle D'Abeau Chesne, BP 701, 38297, Saint Quentin Fallavier) tel que la concentration finale titre 0,1 mM.

Les erlens sont incubés 24h00 supplémentaires sous agitation à température ambiante (20-25°C).
Les cultures ainsi obtenues sont centrifugées (par fraction de 250 ml) à 5000 trs/min pendant 7 minutes, les culots sont repris avec 3 ml de tampon phosphate 10 mM pH = 7,00 (4°C) contenant un mélange d'inhibiteurs de protéases (protease inhibitor cocktail set I 539131, Calbiochem-Novabiochem Corporation, 10394 Pacific center Court, San Diego, CA 92121). Les suspensions bactériennes (regroupées en fractions de 40 ml dans un tube en polypropylène) sont bloquées dans la glace et soumis aux ultrasons (Vibra cell 20 kHz, 72434, Bioblock scientific, Parc d'innovation, BP111, 67403 Illkirch) la sonde étant plongée dans chaque tube 15 secondes (6 chocs de 15 secondes par tube). Chaque tube est centrifugé à 4°C 16000 trs/minutes pendant 1h00.

### i) Purification de la 15-PGDH et de la PGFS.

Les surnageants sont récupérés et introduits dans un tube polypropylène contenant 1 ml de Gluthatione-Sepharose^{r} 4B (40 ml de surnageant pour 1 ml de Gluthatione-Sepharose^{r} 4B) préalablement lavée selon les recommandations du fabricant (Amersham Pharmacia Biotech, 12 avenue des Tropiques, ZA Courtaboeuf, 91944 Les Ulis).
Les tubes sont placés verticalement sur un agitateur rotatif, rotation 10 tours par minutes pendant 1h00 à la température de la pièce (20-25°C), les.
Les tubes sont centrifugés 1000 trs/min pendant 3 minutes, le surnageant est éliminé
Sont introduits dans chacun des tubes 40 ml d'un tampon phosphate 10 mM pH = 7,00. Après agitation douce (retournement) les tubes sont à nouveau centrifugés 3 minutes à 1000 trs/min.
L'opération est réaliseé 5 fois. Un sixième lavage est réalisé avec 40 ml de tampon phosphate salin pH = 7,2 (PBS, Bio-Merieux S , 69280 Marcy-l'Etoile). Après centrifugation le surnageant est à nouveau éliminé.

### j) Elution de la 15-PGDH et de la PGFS.

Une suspension de *thrombin Protease* est reconstituée à 1 unité/µl dans du PBS selon les recommandations du fabricant (Amersham Pharmacia Biotech, 12 avenue des Tropiques, ZA Courtaboeuf, 91944 Les Ulis).
Sont introduits dans chaque tube contenant 1 ml de Gluthatione-Sepharose^{r} 4B, 950 µl de PBS et 50 µl de suspension de thrombine reconstituée. Ceux-ci sont agités en position légéremment inclinée 16h00 à 250 trs/min. Après 16h00 les tubes sont centrifugés à 3000 trs/min pendant 5min, les surnageants sont recueillis. Une évaluation de la quantité de protéine est effectuée en suivant la procédure de dosage Bio-Rad DC Protein Assay (Bio-Rad Laboratories, 2000 Alfred Nobel Dr, Hercules, CA 94547).
Ainsi pour la 15-PGDH comme pour la PGFS on obtient entre 0,2 et 5 mg de protéine par ml, 1 mg /ml le plus souvent.
Les suspensions protéiques ainsi obtenues sont diluées respectivement dans du PBS additionné de 10% de glycérol et du PBS telle que les concentrations protéiques finales titrent 0,2 mg /ml pour la 15-PGDH et 0,5 mg/ml pour la PGFS.
Les suspensions sont bloquées à -80°C jusqu'à utilisation.

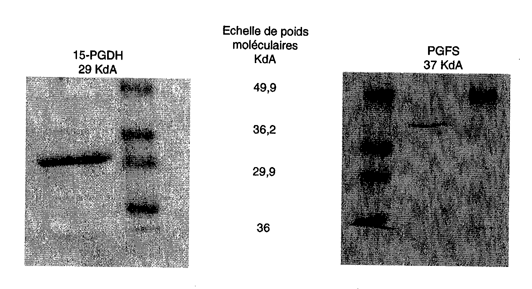

Des analyses électrophorétiques (SDS-Page) réalisées dans des conditions standards démontrent la qualité des résultats ainsi obtenus. Ces résultats sont présentés sur la figure 3.

### Exemple 3: Evaluation de l'effet de molécules sur ces enzymes et caractérisation de certaines familles de molécules comme inhibiteurs spécifiques de la 15 PGDH.

### a) Test 15-PGDH.

L'enzyme obtenue est à la concentration de 0,3 mg/ml et bloquée à - 80°C. Cette suspension est décongélée et stockée dans la glace.
Préparation d'un tampon Tris 100 mM pH = 7,4 contenant 0,1 mM de dithiothreitol (D5545, Sigma-Aldrich, L'isle D'Abeau Chesne, BP 701, 38297, Saint Quentin Fallavier), 1,5 mM de β-NAD (N6522, Sigma-Aldrich, L'isle D'Abeau Chesne, BP 701, 38297, Saint Quentin Fallavier), 50 µM de Prostaglandine E₂ (P4172, Sigma-Aldrich, L'isle D'Abeau Chesne, BP 701, 38297, Saint Quentin Fallavier).
Dans la cuve d'un spectrophotomètre (Perkin-Elmer, Lambda 2) thermostaté à 37°C dont la longueur d'onde de mesure est réglée à 340 nm sont introduits 0,965 ml de ce tampon (préalablement porté à 37°C). 0,035 ml de suspension enzymatique à 37°C sont introduits dans la cuve concommitament à l'enregistrement (augmentation de la densité optique à 340 nm).
La vitesse maximale de réaction est relevée.
Les valeurs essais (molécules) sont comparées à la valeur témoin (sans molécule), les résultats sont exprimés en % de la valeur témoin.

### b) Test PGFS.

L'enzyme obtenue est à la concentration de 0,5 mg/ml et bloquée à - 80°C. Cette suspension est décongélée et stockée dans la glace.
Préparation dans un flacon brun (abri de la lumière) d'un tampon Tris 100 mM pH = 6,5 contenant 20 µM de 9,10 phénanthrène quinone* (P2896, Sigma-Aldrich, L'isle D'Abeau Chesne, BP 701, 38297, Saint Quentin Fallavier) et 100 µM de β-NADPH (N1630, Sigma-Aldrich, L'isle D'Abeau Chesne, BP 701, 38297, Saint Quentin Fallavier).
* Une solution mère titrant 1 mM est préparée dans de l'éthanol absolu portée à 40°C , le flacon est placé dans une cuve à ultrason pour faciliter la solubilisation du produit.
Dans la cuve d'un spectrophotomètre (Perkin-Elmer, Lambda 2) thermostaté à 37°C dont la longueur d'onde de mesure est réglée à 340 nm sont introduits 0,950 ml de ce tampon (préalablement porté à 37°C). 0,05 ml de suspension enzymatique à 37°C sont introduits dans la cuve concomitamment à l'enregistrement (baisse de la densité optique à 340 nm).
La vitesse maximale de réaction est relevée.
Les valeurs essais (molécules) sont comparées à la valeur témoin (sans molécule), les résultats sont exprimés en % de la valeur témoin.

Les résultats obtenus pour les molécules A et B sont les suivants:

| à 50 µM | % Inhibition 15-PGDH | % Inhibition PGFS |
|---|---|---|
| Molecule A : 5-amino-4,6-dichloro-2-phenyl-pyrimidine | 43 | 5 |
| Molécule B : N-[7-(2-Chloro-phenyl)-5-oxo-5,6,7,8-tetrahydro-quinazolin-2-yl]-benzamide | 57 | 10 |

### Exemple 4: mise en évidence de l'activité inhibitrice des composés de formule (I) ou (II)

Les valeurs essais (contenant les composés (I) ou (II)) sont comparées à la valeur témoin (sans composé (I) ou (II)) selon le protocole décrit à l'exemple 3; les résultats indiqués représentent le % de l'inhibition pour une concentration donnée du composé (I) ou (II).

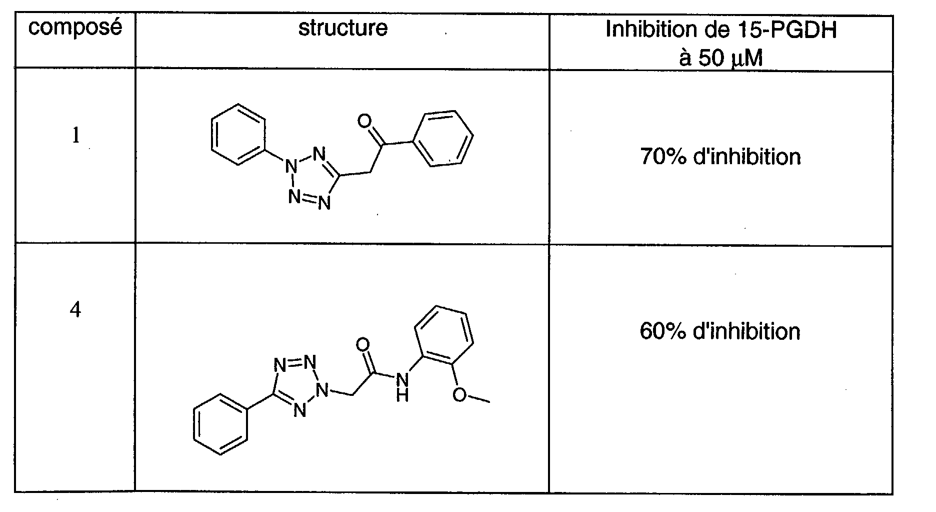

Il ressort de ce tableau que les composés 1 et 4 sont bien des inhibiteurs de 15-PGDH. Ils vont donc permettre une réduction de la chute des cheveux, en particulier naturelle.

### Exemple 5: caractérisation d'inhibiteurs spécifiques de la 15-PGDH

On teste selon le protocole de l'exemple 3, les composés suivants:
- N-(2-methoxyphenyl)-2-[5-phenyl-2H-tetrazol-2-yl]acetamide (composé 4)
- 1-phenyl-2-(2-phenyl-2H-tetrazol-5-yl) ethanone (composé 1)
- N-(4-methylphenyl)-2-{5-[3-(trifluoromethyl)phenyl]-2H-tetrazol-2-yl} acetamide (composé R29A)
Les concentrations des composés testés provoquant une inhibition de 50% respectivement pour les deux enzymes sont rapportées ci-après

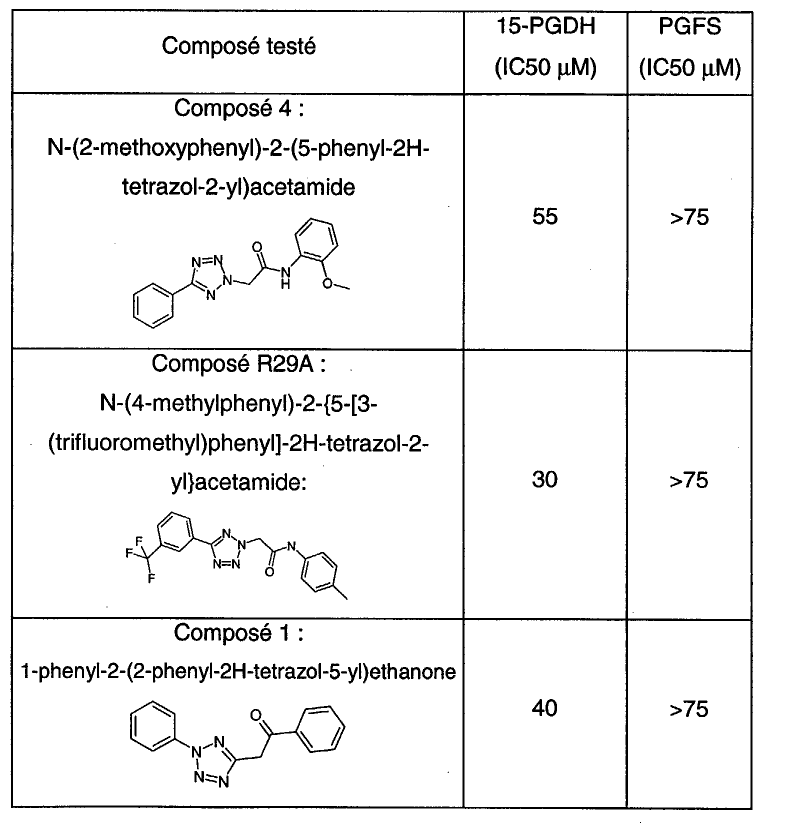

Les composés testés sont des inhibiteurs spécifiques de la 15PGDH. A ce titre, Ils vont donc permettre une réduction de la chute des cheveux, en particulier naturelle.

### Exemple 6.: Mise en évidence de l'efficacité d'un inhibiteur de 15PGDH spécifique sur modèle cellulaire

La présente étude vise à évaluer cette sélection de composés dans un modèle cellulaire. Ceci nous renseignera sur la pénétration de l'actif dans le cytosol et sur son efficacité en tant qu'inhibiteur sélectif de 15-PGDH dans des conditions expérimentales plus complexes qu'un simple milieu réactionnel.

### Matériel et Méthodes

**J-2.** Culture de U937 (CRL-1593 American Type Cells Collection) en milieu RPMI 1640 + 10 % sérum de veau foetal + 2 mM L-Glutamine + Antibiotiques à 37°C sous 5% de CO₂.
**J-1.** Préparation d'une suspension de U937 (1.10⁶ cellules / ml) dans le milieu RPMI 1640 + 10% sérum de veau foetal + 2 mM Glutamine + Antibiotiques + 10 nM PMA (phorbol 12 myristate 13 acetate), introduction de 200 µl/puits de cette suspension dans une boite de 96 puits (3 puits par molécule et par concentration à tester + témoins correspondants). Incubation 36h00 à 37°C sous 5% de CO₂.
**J0.** Elimination des surnageants (les cellules ont adhéré : contrôle microscopique), et introduction dans chaque puits de 100 µl de RPMI 1640 + 2 mM L-Glutamine + 10 ng LPS (sauf témoin absolu) + la molécule à tester à la concentration désirée (ici 5 et 25 µM).
Incubation 6h00 à 37°C sous 5% de CO₂.
Les solutions mères de molécules à tester sont à 25 mM dans le DMSO.
Tous les puits contiendront la même quantité finale de DMSO.

Evaluation immédiate de la quantité de PGF2α sécrétée par les cellules (50 µl) dans les différentes conditions (molécules ou témoins) par l'utilisation d'un kit de dosage immunoenzymatique (Cayman réf. 516011).

### Résultats en % du témoin LPS

| Référence Molécule (5µM) | % du témoin |
|---|---|
| Composé 1 : 1-phenyl-2-(2-phenyl-2H-tetrazol-5-yl)ethanone | + 16 |
| N-(4-methylphenyl)-2-{5-[3-(trifluoromethyl)phenyl]-2H-tetrazol-2-yl}acetamide: | + 46 |
| Composé 4 : N-(2-methoxyphenyl)-2-(5-phenyl-2H-tetrazol-2-yl)acetamide | + 6 |

Cela confirme que les inhibiteurs sélectifs de 15 PGDH sont efficaces dans un environnement cellulaire et protègent les prostaglandines.
Les compositions ci-après sont obtenues par les techniques habituelles couramment utilisées dans le domaine cosmétique ou pharmaceutique.

### EXEMPLE 7 : Lotion capillaire

- Composé 1 0,80 g
- Propylène glycol 10,00 g
- Alcool isopropylique qsp 100,00 g
   On applique cette lotion sur le cuir chevelu, une à deux fois par jour, à raison d'1 ml par application, en massant légèrement le cuir chevelu pour faire pénétrer l'actif. La chevelure est ensuite séchée à l'air libre. Cette lotion permet de diminuer la chute des cheveux et de favoriser leur repousse.

### EXEMPLE 8 : Lotion capillaire

- Composé 4 1,00 g
- Propylène glycol 30,00 g
- Alcool éthylique 40,00 g
- Eau qsp 100,00 g

On applique cette lotion sur le cuir chevelu, une à deux fois par jour, à raison d'1 ml par application, en massant légèrement le cuir chevelu pour faire pénétrer l'actif. La chevelure est ensuite séchée à l'air libre.

## Revendications

1. Utilisation d' au moins un inhibiteur de la 15-hydroxy prostaglandine déshydrogénase (15-PGDH) dans ou pour la préparation d'une composition destinée à stimuler ou induire la croissance et/ou à diminuer la chute et/ou augmenter la densité et/ou réduire l'hétérogénéité du diamètre des fibres kératiniques chez l'être humain..

2. Utilisation selon la revendication 1, **caractérisée en ce que** les fibres kératiniques sont choisies parmi les cheveux, les poils et les cils.

3. Utilisation selon l'une des revendications 1 ou 2, **caractérisée en ce que** l'inhibiteur de la 15-PGDH est un inhibiteur spécifique de la 15-PGDH.

4. Utilisation selon la revendication 3, **caractérisée en ce que** le rapport entre l'activité inhibitrice de la 15-PGDH et l'activité inhibitrice de la prostaglandine F synthase (PGF synthase) est supérieur à 1.

5. Utilisation selon l'une des revendications 1 à 4, **caractérisée en ce que** la composition est une composition cosmétique qui contient en outre des excipients cosmétologiquement acceptables.

6. Utilisation d'au moins un inhibiteur de la 15-PGDH selon l'une des revendications 1 à 5, **caractérisé en ce que** l'inhibiteur est au moins un composé tétrazolique des formule (I) ou (II) ou l'un de ses sels, 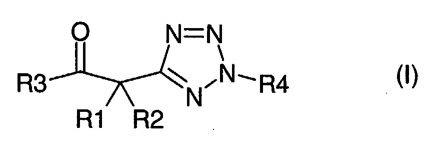 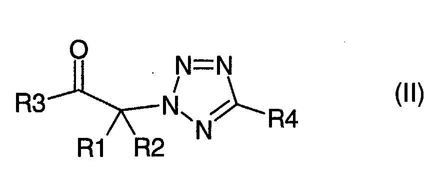 dans lesquelles :
- R₁ et R₂ sont indépendamment choisis parmi l'hydrogène, les halogènes, OR₅, SR₅, NR₅R'₅, COOR₅, COR₅, CONR₅R'₅, CF₃, CN, NR₅COR'₅, SO₂R₅, SO₂NR₅R'₅, NR₅SO₂R'₅, CSR₅, OCOR₅, COSR₅, SCOR₅, CSNR₅R'₅, NR₅CONR'₅R"₅, NR₅C(=NR'₅)NR"₅R"'₅, NR₅CSR'₅, NR₅CSNR'₅R"₅, les radicaux alkyle en C₁-C₂₀, linéaires ou ramifiés, et les cycles de 4 à 7 atomes, contenant éventuellement au moins un hétéroatome, ces cycles pouvant être séparés ou accolés, les radicaux alkyle et les cycles étant, en outre, saturés ou non, et éventuellement substitués par au moins un substituant A1, où R₅, R'₅, R"₅ et R"'₅ désignent indépendamment l'hydrogène, un radical alkyle en C₁-C₂₀, linéaire ou ramifié, ou un cycle hydrocarboné de 4 à 7 atomes, le cycle hydrocarboné ou le radical alkyle étant saturé ou non et éventuellement substitué par au moins un substituant A2 ;
- R₃ est choisi parmi l'hydrogène, OR₆, SR₆, NR₆R'₆, CF₃, NR₆COR'₆, NR₆SO₂R'₆, NR₆CONR'₆R"₆, NR₆CSR'₆, NR₆CSNR'₆R"₆, les radicaux alkyle en C₁-C₂₀, linéaires ou ramifiés, et les cycles hydrocarbonés de 4 à 7 atomes, séparés ou accolés, les radicaux alkyle et les cycles hydrocarbonés étant, en outre, saturés ou non et éventuellement substitués par au moins un substituant A₃, avec R₆, R'₆ et R"₆ désignant indépendamment l'hydrogène, un radical alkyle en C₁-C₂₀, linéaire ou ramifié, ou un cycle hydrocarboné de 4 à 7 atomes, le radical alkyle ou le cycle hydrocarboné étant saturé ou non et éventuellement substitué par au moins un substituant A₄ ;
- R₄ est choisi parmi l'hydrogène, COOR₇, CONR₇R'₇, SO₂R₇, SO₂NR₇R'₇, COR₇, CSR₇, COSR₇, CSNR₇R'₇, les radicaux alkyle en C₁-C₂₀, linéaires ou ramifiés, et les cycles hydrocarbonés de 4 à 7 atomes, séparés ou accolés, les radicaux alkyle et les cycles hydrocarbonés étant, en outre, saturés ou non et éventuellement substitués par au moins un substituant A₅ ; R₄ peut, en outre, représenter, dans le cas de la formule (II), un halogène, OR₇, SR₇, NR₇R'₇, CF₃, CN, NR₇COR'₇, NR₇SO₂R'₇, OCOR₇, SCOR₇, NR₇CONR'₇R"₇, NR₇C(=NR'₇)NR"₇R"'₇, NR₇CSR'₇ ou NR₇CSNR'₇R"₇, avec R₇, R'₇, R"₇ et R"'₇ désignant indépendamment l'hydrogène, un radical alkyle en C₁-C₂₀, linéaire ou ramifié, ou un cycle hydrocarboné de 4 à 7 atomes, le radical alkyle ou le cycle hydrocarboné étant saturé ou non et éventuellement substitué par au moins un substituant A₆ ;
- A₁ et A₂ sont indépendamment choisis parmi les halogènes, les hétérocycles comportant de 4 à 7 atomes et au moins un hétéroatome, OR₈, SR₈, NR₈R'₈, COOR₈, CONR₈R'₈, CF₃, CN, NR₈COR'₈, SO₂R₈, SO₂NR₈R'₈, NR₈SO₂R'₈, COR₈, CSR₈, OCOR₈, COSR₈, SCOR₈, CSNR₈R'₈, NR₈CONR'₈R"₈, NR₈C(=NR'₈)NR"₈R"'₈, NR₈CSR'₈, NR₈CSNR'₈R"₈ ;
- A₃ et A₄ sont indépendamment choisis parmi les halogènes, R₉, OR₉, SR₉, NR₉R'₉, COOR₉, CONR₉R'₉, CF₃, CN, NR₉COR'₉, SO₂R₉, SO₂NR₉R'₉, NR₉SO₂R'₉, CSR₉, OCOR₉, COSR₉, SCOR₉, CSNR₉R'₉, NR₉CONR'₉R"₉, NR₉C(=NR'₉)NR"₉R"'₉, NR₉CSR'₉, NR₉CSNR'₉R"₉ ;
- A₅ et A₆ sont indépendamment choisis parmi les halogènes, R₁₀, OR₁₀, SR₁₀, NR₁₀R'₁₀, CF₃, CN, NR₁₀COR'₁₀, SO₂R₁₀, SO₂NR₁₀R'₁₀, NR₁₀SO₂R'₁₀, CSR₁₀, OCOR₁₀, SCOR₁₀, CSNR₁₀R'₁₀, NR₁₀CONR'₁₀R"₁₀, NR₁₀C(=NR'₁₀)NR"₁₀R"'₁₀, NR₁₀CSR'₁₀, NR₁₀CSNR'₁₀R"₁₀;
- R₈, R'₈, R"₈, R"'₈, R₉, R'₉, R"₉, R"'₉, R₁₀, R'₁₀, R"₁₀ et R"'₁₀ désignant indépendamment l'hydrogène, un radical alkyle en C₁-C₂₀, linéaire ou ramifié, saturé ou non, un cycle hydrocarboné de 4 à 7 atomes, saturé ou non, ou un radical benzyle.

7. Utilisation d'au moins un composé de formule (I) ou (II) selon la revendication 6, **caractérisé en ce que**
- R₁ et R₂ sont indépendamment choisis parmi l'hydrogène, les halogènes, OR₅, SR₅, NR₅R'₅, COOR₅, CF₃, CN, les radicaux alkyle en C₁-C₂₀, linéaires ou ramifiés, et les cycles de 4 à 7 atomes, contenant éventuellement au moins un hétéroatome, ces cycles pouvant être séparés ou accolés, les radicaux alkyle et les cycles étant, en outre, saturés ou non, où R₅ et R'₅ désignent indépendamment l'hydrogène, un radical alkyle en C₁-C₂₀, linéaire ou ramifié, ou un cycle hydrocarboné de 4 à 7 atomes, le cycle hydrocarboné ou le radical alkyle étant saturé ou non ;
- R₃ est choisi parmi l'hydrogène, OR₆, SR₆, NR₆R'₆, CF₃, les radicaux alkyle en C₁-C₂₀, linéaires ou ramifiés, et les cycles hydrocarbonés de 4 à 7 atomes, séparés ou accolés, les radicaux alkyle et les cycles hydrocarbonés étant, en outre, saturés ou non et éventuellement substitués par au moins un substituant A₃, avec R₆ et R'₆ désignant indépendamment l'hydrogène, un radical alkyle en C₁-C₂₀, linéaire ou ramifié, ou un cycle hydrocarboné de 4 à 7 atomes, le radical alkyle ou le cycle hydrocarboné étant saturé ou non et éventuellement substitué par au moins un substituant A₄ ;
- R₄ est choisi parmi l'hydrogène, COOR₇, CSR₇, les radicaux alkyle en C₁-C₂₀, linéaires ou ramifiés, et les cycles hydrocarbonés de 4 à 7 atomes, séparés ou accolés, les radicaux alkyle et les cycles hydrocarbonés étant, en outre, saturés ou non ; R₄ peut, en outre, représenter, dans le cas de la formule (II), un halogène, OR₇, SR₇, NR₇R'₇, CF₃, CN , avec R₇ et R'₇, désignant indépendamment l'hydrogène, un radical alkyle en C₁-C₂₀, linéaire ou ramifié, ou un cycle hydrocarboné de 4 à 7 atomes, le radical alkyle ou le cycle hydrocarboné étant saturé ou non, éventuellement substitué par au moins un substituant choisi parmi OR₁₀, SR₁₀, NR₁₀R'₁₀, CF₃, avec R10 et R'10 désignant un radical alkyle en C₁-C₂₀ linéaire ou ramifié, saturé ou non;
- A₃ et A₄ sont indépendamment choisis parmi les halogènes, R₉, OR₉, SR₉, NR₉R'₉, COOR₉, CF₃, avec R₉ et R'₉, désignant indépendamment l'hydrogène, un radical alkyle en C₁-C₂₀, linéaire ou ramifié, saturé ou non, un cycle hydrocarboné de 4 à 7 atomes, saturé ou non, ou un radical benzyle.

8. Utilisation d'au moins un composé selon la revendication 6 **caractérisé en ce que** dans la formule (I) ou (II)
- R₃ est choisi parmi l'hydrogène, OR₆, SR₆, NR₆R'₆, CF₃, les radicaux alkyle en C₁-C₂₀, linéaires ou ramifiés, et les cycles hydrocarbonés de 4 à 7 atomes, séparés ou accolés, les radicaux alkyle et les cycles hydrocarbonés étant, en outre, saturés ou non et éventuellement substitués par au moins un substituant A₃, avec R₆ et R'₆ désignant indépendamment l'hydrogène, un radical alkyle en C₁-C₂₀, linéaire ou ramifié, ou un cycle hydrocarboné de 4 à 7 atomes, le radical alkyle ou le cycle hydrocarboné étant saturé ou non et éventuellement substitué par au moins un substituant A₄ ;
- R₄ est choisi parmi l'hydrogène, les radicaux alkyle en C₁-C₂₀, linéaires ou ramifiés, et les cycles hydrocarbonés de 4 à 7 atomes, séparés ou accolés, les radicaux alkyle et les cycles hydrocarbonés étant, en outre, saturés ou non éventuellement substitué par au moins un substituant choisi parmi OR₁₀, SR₁₀, NR₁₀R'₁₀, CF₃, avec R10 et R'10 désignant un radical alkyle en C₁-C₂₀ linéaire ou ramifié, saturé ou non;
- A₃ et A₄ sont indépendamment choisis parmi R₉, OR₉, SR₉, NR₉R'₉, COOR₉, avec R₉ et R'₉, désignant indépendamment l'hydrogène, un radical alkyle en C₁-C₂₀, linéaire ou ramifié, saturé ou non, un cycle hydrocarboné de 4 à 7 atomes, saturé ou non, ou un radical benzyle.

9. Utilisation selon l'une des revendications 6 à 8 **caractérisée en ce que** l'un au moins des R₁ et R₂ représentent un atome d'hydrogène ou un atome d'halogène.

10. Utilisation selon l'une des revendications 6 à 9, **caractérisée en ce que** R₃ représente NR₆R'₆ ou un radical aryle, éventuellement substitué par le substituant A₃.

11. Utilisation selon l'une des revendications 6 à 10, **caractérisée en ce que** R₃ représente un radical naphtyle ou un radical phényle, éventuellement substitué par OR₉.

12. Utilisation selon la revendication précédente, **caractérisée en ce que** R₄ représente un radical aryle et notamment un radical naphtyle ou un radical phényle.

13. Utilisation selon l'une des revendications 6 à 12, **caractérisée en ce que** R₆ représente l'hydrogène et R'₆ un radical aryle, en particulier phényle, éventuellement substitué par le groupe OR₉.

14. Utilisation selon l'une des revendications 6 à 13, **caractérisée en ce que** R₉ représente un radical alkyle en C₁-C₂₀ et mieux en C₁-C₁₀, linéaire ou ramifié, saturé.

15. Utilisation selon l'une des revendications 6 à 14, **caractérisée en ce que** le sel du composé de formule (I) ou (II) est un sel choisi parmi les sels de sodium, de potassium, les sels de zinc (Zn²⁺), de calcium (Ca²⁺), de cuivre (Cu²⁺), de fer (Fe²⁺), de strontium (Sr²⁺), de magnésium (Mg²⁺), de manganèse (Mn²⁺), les sels de tri-éthanolamine, mono-éthanolamine, di-éthanolamine, hexadécylamine, N,N,N',N'-tétrakis-(hydroxypropyl-2) éthylène di-amine, tris-hydroxyméthyl aminométhane, les hydroxydes et les carbonates.

16. Utilisation selon l'une des revendications 6 à 15, **caractérisée en ce que** le composé de formule (I) ou (II) est choisi parmi :
1-phenyl-2-(2-phenyl-2H-tetrazol-5-yl)ethanone: 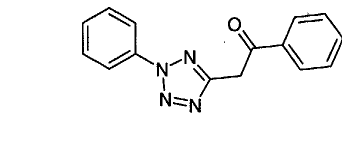
1-(2-methoxyphenyl)-2-(2-phenyl-2H-tetrazol-5-yl)ethanone 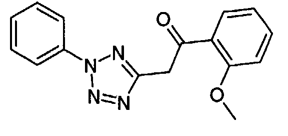
N-(4-methylphenyl)-2-{5-[3-(trifluoromethyl)phenyl]-2H-tetrazol-2-yl}acetamide: 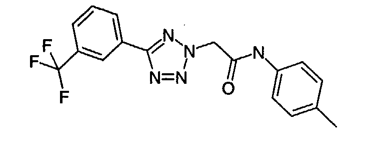
N-(2-phenyl)-2-(5-phenyl-2H-tetrazol-2-yl)acetamide 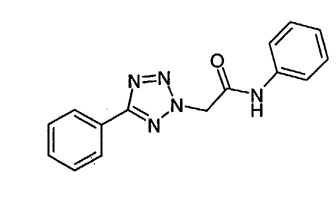
N-(2-methoxyphenyl)-2-(5-phenyl-2H-tetrazol-2-yl)acetamide:

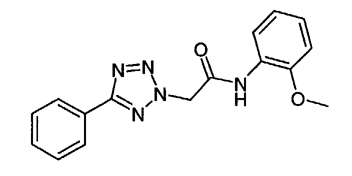

17. Utilisation selon l'une des revendications 1 à 16, **caractérisée en ce que** la composition est destinée à la voie orale.

18. Utilisation selon l'une des revendications 1 à 16, **caractérisé en ce que** la composition est une composition cosmétique ou dermatologique destinée à une application par la voie topique.

19. Utilisation selon la revendication 18, **caractérisé en ce que** la composition est destinée à une application sur le cuir chevelu.

20. Utilisation selon l'une des revendications 1 à 19, **caractérisé en ce que** l'inhibiteur de 15-PGDH est présent à une concentration allant de 10⁻³ à 5%, par rapport au poids total de la composition.

21. Composition cosmétique ou pharmaceutique, contenant au moins un inhibiteur de 15-hydroxyprostaglandine déshydrogénase (15-PGDH) tel que défini dans au moins une des revendications 1 à 20, et des excipients physiologiquement acceptables.

22. Composition selon la revendication 21, **caractérisée en ce que** l'inhibiteur de 15-PGDH est un composé de formule (I) ou (II), tel que défini selon l'une des revendications 6 à 16.

23. Composition selon l'une des revendications 21 ou 22, **caractérisé en ce que** l'inhibiteur de 15-PGDH est un inhibiteur spécifique de 15-PGDH, qui est peu ou pas inhibiteur de PGFS.

24. Composition selon au moins l'une des revendications 21 à 23, **caractérisé en ce que** l'inhibiteur de 15-PGDH est choisi parmi
Molécule A:
5-amino-4,6-dichloro-2-phenyl-pyrimidine 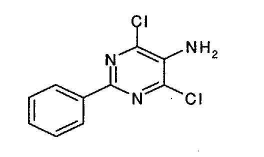
Molécule B:
N-[7-(2-Chloro-phenyl)-5-oxo-5,6,7,8-tetrahydro-quinazolin-2-yl]-benzamide 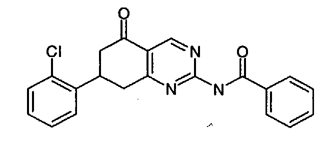 1-phenyl-2-(2-phenyl-2H-tetrazol-5-yl)ethanone: 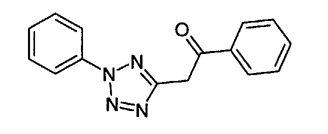 N-(4-methylphenyl)-2-{5-[3-(trifluoromethyl)phenyl]-2H-tetrazol-2-yl}acetamide: 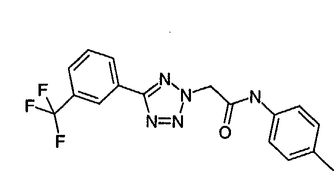 N-(2-methoxyphenyl)-2-(5-phenyl-2H-tetrazol-2-yl)acetamide:

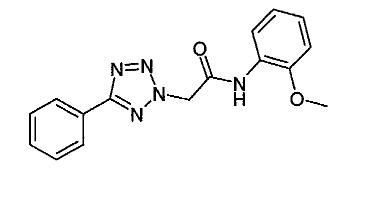

25. Composition selon l'une des revendications 21 à 24, **caractérisée en ce que** l'inhibiteur de 15-PGDH est présent à une concentration de 0,001% à 5% p/v.

26. Composition selon l'une des revendications 21 à 25, **caractérisée en ce qu'**elle comprend un milieu cosmétologiquement acceptable constitué d'eau ou d'au moins un solvant choisi parmi les solvants organiques hydrophiles, les solvants organiques lipophiles, les solvants organiques amphiphiles et leurs mélanges.

27. Composition selon l'une au moins des revendications 21 à 26, **caractérisé en ce qu'**au moins un inhibiteur de la 15-hydroxy prostaglandine déshydrogénase est encapsulé dans une structure choisie parmi les microsphères, les nanosphères, les oléosomes ou les nanocapsules.

28. Composition selon l'une des revendications 21 à 27, **caractérisé en ce qu'**elle contient d'autres ingrédients choisis parmi les solvants, les épaississants ou gélifiants de phase aqueuse ou de phase huileuse, les matières colorantes solubles dans le milieu de la composition, les charges, les pigments, les antioxydants, les conservateurs, les parfums, les électrolytes, les neutralisants, les agents bloqueurs d'U.V., les actifs cosmétiques et pharmaceutiques, leurs mélanges.

29. Composition selon au moins l'une des revendications 21 à 28 **caractérisé en ce qu'**elle contient en outre au moins un agent accélérateur de pénétration.

30. Composition selon au moins l'une des revendications 21 à 29, **caractérisée en ce qu'**elle comprend en outre au moins un agent actif pour favoriser la croissance et/ou retarder ou empêcher la chute des cheveux qui n'est pas un inhibiteur sélectif de la 15-PGDH.

31. Composition selon au moins l'une des revendications 21 à 30, **caractérisé en ce qu'**elle comprend en outre au moins une prostaglandine et/ou un dérivé et/ou un analogue de prostaglandine.

32. Composition selon la revendication 31, **caractérisé en ce qu'**elle comprend au moins un composé choisi parmi les prostaglandines de la série 2 dont notamment PGF 2 α et PGE 2 sous forme salines ou sous forme de précurseurs, notamment des esters tels que les isopropyl esters, le 16,16 dimethyl PGE2, le 17 phényl PGE2, le 16,16 diméthyle PGF2α, le 17 phényl PGF2α, les prostaglandines de la série 1 tel que le 11 déoxy prostaglandine E1, le 1 déoxy prostaglandine E1 sous forme salines ou esters, le latanoprost, le travoprost, le fluprostenol, l'unoprostone, le bimatoprost, le cloprosténol, le viprostol, le butaprost, le misoprostol, cicaprost, l'iloprost, l'isocarbacycline, le beraprost, leurs sels ou leurs esters, les esters du récepteur de la prostaglandine D2 tel le BW245C ((4S)-(3-[(3R,S)-3-cyclohexyl-3-isopropyl]-2,5-dioxo)4-imidazolidine-heptanoic acid), le BW246C ((4R)-(3-[(3R,S)-3-cyclohexyl-3-isopropyl]-2,5-dioxo)4-imidazolidineheptanoic acid), les esters du récepteur aux thromboxanes A2 (TP) tel le I-BOP ([1S-[1a,2a(Z), 3b(1E,3S),4a]]-7-[3-[3-hydroxy-4-[4-(iodophenoxy)-1-butenyl]-7-oxabicyclo[2.2.1]hept-2-yl]-5-heptenoic acid), ou leurs mélanges.

33. Composition selon l'une au moins des revendications 21 à 32, **caractérisée en ce qu'**elle comprend en outre au moins un agoniste prostanoïque ou non prostanoïque des récepteurs EP2 et/ou EP4.

34. Procédé de traitement cosmétique pour maintenir et /ou augmenter la densité des fibres kératiniques et/ou empêcher et/ou retarder la chute des fibres kératiniques chez l'être humain, **caractérisé en ce qu'**il comprend l'administration d'au moins un inhibiteur de la 15-hydroxy prostaglandine déshydrogénase tel que défini dans l'une des revendications précédentes, ou d'une composition selon l'une des revendications 21 à 33 ou susceptible d'être préparée selon l'une des revendications 1 à 20.

35. Procédé de traitement cosmétique selon la revendication34, **caractérisé en ce que** les fibres kératiniques sont choisi parmi les cheveux et les cils.

36. Procédé de traitement cosmétique selon l'une des revendications 34 ou 35, **caractérisé en ce que** l'administration de l'inhibiteur de 15-PGDH ou de la composition le contenant est effectuée par voie orale.

37. Procédé de traitement cosmétique selon l'une des revendications 34 ou 35, **caractérisé en ce que** l'inhibiteur de 15-PGDH ou la composition le contenant sont appliqués par voie topique, sur les cheveux et/ou le cuir chevelu et/ou les cils.
